# EUROPEAN PATENT SPECIFICATION

(11) **EP 3 482 627 B1**
(45) Date of publication and mention of the grant of the patent: **29.07.2020**
(21) Application number: 18210158.4
(22) Date of filing: 07.01.2011
(51) Int. Cl.: A01H 15/00, C07H 21/04, C12Q 1/25, A01H 17/00, C12N 1/00, C12N 1/14, C12Q 1/68, A01H 3/00

(54) **ENDOPHYTES AND RELATED METHODS**
ENDOPHYTEN UND ENTSPRECHENDE VERFAHREN
ENDOPHYTES ET PROCÉDÉS APPARENTÉS

(30) Priority: 07.01.2010 AU 2010900054; 25.06.2010 AU 2010902821
(43) Date of publication of application: 15.05.2019
(62) Divisional of application: 15190449.7
(73) Proprietor: Agriculture Victoria Services Pty Ltd, Bundoora, VIC 3083 (AU)
(72) Inventor: Spangenberg, German, Bundoora, Victoria 3083 (AU); Guthridge, Kathryn Michaela, Hadfield, Victoria 3046 (AU); Forster, John White, Diamond Creek, Victoria 3089 (AU); Sawbridge, Timothy Ivor, Coburg, Victoria 3058 (AU); Ludlow, Emma Jane Isobel, Viewbank, Victoria 3084 (AU); Kaur, Jatinder, Taylors Hill, Victoria 3037 (AU); Rochfort, Simone Jane, Reservoir, Victoria 3073 (AU); Rabinovich, Maia Andrea, 8000 Buenos Aires (AR); Ekanayake, Piyumi, Bundoora, Victoria 3083 (AU)
(74) Representative: Grund, Martin

(56) References cited:
- WO-A1-2004/029227
- WO-A2-2004/106487
- NZ-A- 233 083
- US-A- 6 072 107
- CHRISTENSEN M J ET AL: "Taxonomy of Acremonium endophytes of tall fescue (Festuca arundinacea), meadow fescue (Festuca pratensis) and perennial rye-grass (Lolium perenne)", MYCOLOGICAL RESEARCH, ELSEVIER, GB, vol. 97, no. 9, 1 January 1993 (1993-01-01), pages 1083-1092, XP008100497, ISSN: 0953-7562, DOI: 10.1016/S0953-7562(09)80509-1
- DE JONG ELINE VAN ZIJLL ET AL: "Global genetic diversity of the perennial ryegrass fungal endophyte Neotyphodium lolii", CROP SCIENCE, vol. 48, no. 4, July 2008 (2008-07), pages 1487-1501, XP009188759, ISSN: 0011-183X
- YOUNG C A ET AL: "A complex gene cluster for indole-diterpene biosynthesis in the grass endophyte Neotyphodium lolii", FUNGAL GENETICS AND BIOLOGY, SAN DIEGO, CA, US, vol. 43, no. 10, 1 October 2006 (2006-10-01), pages 679-693, XP024918970, ISSN: 1087-1845, DOI: 10.1016/J.FGB.2006.04.004 [retrieved on 2006-10-01]

### SEQUENCE LISTING

<110> Agriculture Victoria Services Pty Ltd
<120> Endophytes and related methods
<130> 213-003T1T1
<140>
   <141> 2011-01-07
<150> PCT/AU2011/000020
   <151> 2011-01-07
<150> AU2010900054
   <151> 2010-01-07
<150> AU2010902821
   <151> 2010-06-25
<160> 21
<170> PatentIn version 3.5
<210> 1
   <211> 124
   <212> DNA
   <213> Epichloe festucae E2368
<400> 1
   gcaggggaat gaggcgttcg gctgtgatga cggtggctgt tgaaaccgcc gcatgtttgc 60
   tactgcgtac gtacttttgg tgtttctcca acatagtaac tgtttcaata ttagaatgga 120
   tgta 124
<210> 2
   <211> 104
   <212> DNA
   <213> Endophyte E1
<400> 2
<210> 3
   <211> 63
   <212> DNA
   <213> Endophyte NEA12
<400> 3
   gcagggggaa tgaggcgttc ggctgtgatg atggtggctg ttgaaaccgc cgcatgtttg 60
   cta 63
<210> 4
   <211> 62
   <212> DNA
   <213> Neotyphodium lolii
<400> 4
   gcaggggaat gaggcgttcg gctgtgatga cggtggctgt tgaaaccgcc gcatgtttgc 60
   ta 62
<210> 5
   <211> 125
   <212> DNA
   <213> Epichloe festucae E2368
<400> 5
   gccatgaaag catttaatgg tcgcttagct ttttcgcaaa tctgaacttt tgatttcaga 60
   tgagagttca ttgtcgtttg gttgaatgtc ggcgtcatag tgggctgaaa tgtagtgtcg 120
   aaata 125
<210> 6
   <211> 125
   <212> DNA
   <213> Endophyte E1
<400> 6
   gccatgaaag catttaatgg tcgcttagct ttttcgcaag tctgaacttt tgatttcaga 60
   tgagagttca ttgtcgtttg gttgaatgtc ggcgtcatag tgggctgaaa tgtagtgtcg 120
   aaata 125
<210> 7
   <211> 106
   <212> DNA
   <213> Endophyte NEA12
<400> 7
   ttatggccaa taccgatttg ctgtgcaaga ctctcacgca aatttggagg ctggtctcaa 60
   atattttaaa tgttcaaatt gatgtttcag ctatgtagcg taaact 106
<210> 8
   <211> 105
   <212> DNA
   <213> Neotyphodium lolii
<400> 8
   tatggccaat accgatttgc tgtgcaagac tctcacgcaa atttggaggc tggtctcaaa 60
   tattttaaat gttcaaattg atgtttcagc tatgtagcgt aaact 105
<210> 9
   <211> 125
   <212> DNA
   <213> Epichloe festucae E2368
<400> 9
   gcggagaaaa ccaaataggt cccgaatacc agagcaacta tggctcctga ctcgaagttg 60
   atgaattgga gggcgaaact cacagggttt cagttgctgc atgtctctga attagattct 120
   ctatt 125
<210> 10
   <211> 125
   <212> DNA
   <213> Endophyte E1
<400> 10
   gcggagaaaa ccaaataggt cccgaatacc agagcaacta tggctcctga ctcgaagttg 60
   atgaattgga gggcgaaact cacagggttt cagttgctgc atgtctctga attagattct 120
   ctatt 125
<210> 11
   <211> 30
   <212> DNA
   <213> Endophyte NEA12
<400> 11
   gagaattttc tttcaattgt acaaagcgcg 30
<210> 12
   <211> 125
   <212> DNA
   <213> Neotyphodium lolii
<400> 12
   gagaattttc tttcaattgt acaaagcgcg gaaacgtagc gacgaccgcc gcggggatca 60
   atggagttat gggcgcatta cataggctct cacaagttgc atgtctctga attagattct 120
   ctatt 125
<210> 13
   <211> 539
   <212> DNA
   <213> Epichloe festucae
<400> 13
   accagacgat acaatctcga tagtaaccgc ctgccccata gcgggattga ttacacccag 60
   gcagctcatg acagtggtat caatctccaa tataagaccc tacgaacgga ctctgatata 120
   acgccatcgt ccccgactca tgatgcccat gtgaaacctt taccagttgc caacgccgtg 180
   tcctcgttag aggtcctgaa caatctgtgt gaacagagta gttggaaatg ggtggaaggt 240
   atgttaattg gaggctgtct tcaatacggc ctagagcgat acgatgatgc gttcaagtcc 300
   ttctcaagga ttgtcgcagt tgattccagg taagttgctc gccacaatac cctcactcct 360
   ctgcttgatc tcacaatcac cggcttccca gccatgttga agctatcagt catatgggcg 420
   cagccttgta ttgcctcgga cgtcaagatg aagcagagaa aattggctcc gggtgataaa 480
   gctacgacca aattatctcg atgccacgga acacttggtg ggccatcttt ataaaaatc 539
<210> 14
   <211> 540
   <212> DNA
   <213> Endophyte NEA11
<400> 14
   accagacgat acaatctcga tagtaaccgc ctgccccata gcgggattga ttacacccag 60
   gcagctcatg acagtggtat caatctccaa tataagaccc tacgaacgga ctctgatata 120
   acgccatcgt ccccgactca tgatgcccat gtgaaacctt taccagttgc caacgccgtg 180
   tcctcgttag aggtcctgaa caatctgtgt gaacagagta gttggaaatg ggtggaaggt 240
   atgttaattg gaggctgtct tcaatacggc ctagagcgat acgatgatgc gttcaagtcc 300
   ttctcaagga ttgtcgcagt tgattccagg taagttgctc gccacaatac cctcactcct 360
   ctgcttgatc tcacaatcac cggcttccca gccatgttga agctatcagt catatgggcg 420
   cagccttgta ttgcctcgga cgtcaagatg aagcagagaa aaaattggct ccgggtgata 480
   aagctacgac caaattatct cgatgccacg gaacacttgg tgggccatct ttataaaaat 540
<210> 15
   <211> 473
   <212> DNA
   <213> Endophyte NEA11
<400> 15
   atagcggaat tgattacagc caggcagttc attacagtgg tatcaatctc caatataaga 60
   ccacacgaac ggactctgat gtaacgccat cgtccccgac tcatgatgcc catgtgaagc 120
   ctttacccgt cgccaacgcc gtgtcttcgc tagaggtcct gaacaacctg tgtgaacaga 180
   gtggttggaa atgggtggaa ggtatgttag ttggaggctg tcttcagtac ggtctagagc 240
   agtacgaaga tgcgttcaag tccttctcaa ggattgtcgc agttgattca aggtaagctg 300
   cccgccacaa tactcccatt ccgttgcttg atctcacgat ccctggcttc ctagccatgt 360
   tgaagctatc agtcatatgg gcgcagcctt gtattgcctc ggacgccaag acgaagcaga 420
   gcaaaattgg ctccgggtgg taaagctacg accaaactat ctcgatgcca cgg 473
<210> 16
   <211> 300
   <212> DNA
   <213> Neotyphodium lolii
<400> 16
   gcgcgtcacg atttcccatt taacaccctc agtcacgcgg ctgatagacc cagattcaca 60
   accttttcta aagacgatgg tgtttaccgg cgagcctctg tctgtggacg atgctacccg 120
   atggtgggga aaggtcgacg tcgtcaacga atatgggcct gacgagtgca ccatcaacac 180
   tgtcaacagc cgacctatca gtcctgaagc tgctacgaac atagggctgc cggttggagt 240
   ggccgcttgg attaccgacc cggaaaacca tcaagtactc gttccgatcg gctgtgttgg 300
<210> 17
   <211> 266
   <212> DNA
   <213> Endophyte NEA11
<400> 17
   gcgcgtcacg attgcccatt taacaccctc agtcacgcga ctgatagacc cagattcaca 60
   acccgtcctc aagacgatgg tgtttaccgg cgagcctctg tctgtggacg atgctacccg 120
   atggtgggga aaggtcgacg tcgtcaacga atatgggctg cagagtgcac catcaacact 180
   gtcaacagcc gacctatcag tcctgaagcc gctgcgaaca tagggctgcc ggttggagtg 240
   gccgcttgga ttacagaccc ggaaaa 266
<210> 18
   <211> 255
   <212> DNA
   <213> Endophyte NEA11
<400> 18
   gcgcgtcacg attgcccatt taacaccctc agtcacgcga ctgatagacc cagattcaca 60
   acccgtcctc aagacgatgg tgtttaccgg cgagcctctg tctgtggacg atgctacccg 120
   atggtgggga aagttcgacg tcgtcaacga atatgggcct gcagagtgca ccatcaacac 180
   tgtcaacagc cgacctatca gtcctgaagc cgctgcgaac atagggctgc cggttggagt 240
   ggccgcttgg attac 255
<210> 19
   <211> 53
   <212> DNA
   <213> Endophyte NEA11
<400> 19
   gcgcgtcacg attgcccatt taacaccctc agtcacgcga ctgatagacc cag 53
<210> 20
   <211> 85
   <212> DNA
   <213> Endophyte NEA11
<400> 20
   gcgcgtcacg attgcccatt taacaccctc agtcacgcga ctgatagacc cagattcaca 60
   acccgtcctc aagacgatgg tgttt 85
<210> 21
   <211> 44
   <212> DNA
   <213> Endophyte NEA11
<400> 21
   gacccggaaa atgatcaagt actcgttcca atcggctgtg ttgg 44

## Description

### Field of the invention

The present invention relates to endophytic fungi (endophytes) and nucleic acids thereof, to plants infected with endophytes and to related methods, including methods of selecting, breeding and/or characterising endophytes.

### Background of the invention

Important forage grasses perennial ryegrass and tall fescue are commonly found in association with fungal endophytes.

Both beneficial and detrimental agronomic properties result from the association, including improved tolerance to water and nutrient stress and resistance to insect pests.

Insect resistance is provided by specific metabolites produced by the endophyte, in particular loline alkaloids and peramine. Other metabolites produced by the endophyte, lolitrems and ergot alkaloids, are toxic to grazing animals and reduce herbivore feeding.

Considerable variation is known to exist in the metabolite profile of endophytes. Endophyte strains that lack either or both of the animal toxins have been introduced into commercial cultivars.

Molecular genetic markers such as simple sequence repeat (SSR) markers have been developed as diagnostic tests to distinguish between endophyte taxa and detect genetic variation within taxa. The markers may be used to discriminate endophyte strains with different toxin profiles.

However, there remains a need for methods of identifying, isolating and/or characterising endophytes and a need for new endophyte strains having desired properties.

In the fungal kingdom, there is no differentiation of individuals into sexes generating different gametes, but instead mating-type identity is determined by inheritance of alleles at specific mating-type loci.

The mating-type (*MAT*) genes constitute master regulators of sexual reproduction in filamentous fungi. Although mating-type loci consist of one to a few linked genes, and are thus limited to a small genomic region, alternate sequences at *MAT,* denoted idiomorphs, lack significant sequence similarity and encode different transcriptional regulators.

Fusion events are required during sexual reproduction in filamentous ascomycete species. Although cell fusion processes associated with vegetative growth as opposed to sexual development serve different developmental functions, both require extracellular communication and chemotropic interactions, followed by cell wall breakdown, membrane-merger and pore formation.

A number of genes have been characterised that are required for both sexual reproduction and vegetative hyphal fusion, including components of the MAPK pathway which is activated in response to pheromone perception during mating. The expression of pheromone precursors and pheromone receptor genes is directly controlled by transcription factors encoded by the mating-type genes.

Hyphal fusion occurs readily within an individual colony during vegetative growth, maintaining the physiological continuity of the organism. Hyphal fusion between different endophyte strains of opposite mating-type may be promoted by treating the mycelia with a combination of cell wall-degrading enzymes and fusion agents such as PEG4000.

However, there remains a need for methods of molecular breeding of endophytes and for new endophyte strains having desired properties.

*Neotyphodium* endophytes are not only of interest in agriculture, as they are a potential source for bioactive molecules such as insecticides, fungicides, other biocides and bioprotectants, allelochemicals, medicines and nutraceuticals.

Difficulties in artificially breeding of these endophytes limit their usefulness. For example, many of the novel endophytes known to be beneficial to pasture-based agriculture exhibit low inoculation frequencies and are less stable in elite germplasm. Thus, there remains a need for methods of molecular breeding of novel, highly compatible endophytes.

It is an object of the present invention to overcome, or at least alleviate, one or more of the difficulties or deficiencies associated with the prior art.

The invention is defined by the appended claims.

Disclosed is a method for selecting and/or characterising an endophyte strain, said method including:
providing a plurality of samples of endophytes;
subjecting said endophytes to genetic analysis;
subjecting said endophytes to metabolic analysis; and
selecting endophytes having a desired genetic and metabolic profile.

The method may include the further step of assessing geographic origin of the endophytes and selecting endophytes having a desired genetic and metabolic profile and a desired geographic origin.

The plurality of samples of endophytes may be provided by a method including:
providing a plurality of plant samples; and
isolating endophytes from said plant samples.

The method may be performed using an electronic device, such as a computer.

Applicant has surprisingly found that specific detection of endophytes *in planta* with markers such as SSR markers has provided the tools for efficient assessment of endophyte genetic diversity in diverse grass populations and the potential discovery of novel endophyte strains.

A large scale endophyte discovery program was undertaken to establish a 'library' of novel endophyte strains. A collection of perennial ryegrass and tall fescue accessions was established.

Genetic analysis of endophytes in these accessions has lead to the identification of a number of novel endophyte strains. These novel endophyte strains are genetically distinct from known endophyte strains.

Metabolic profiling was undertaken to determine the toxin profile of these strains.

Specific detection of endophytes *in planta* with SSR markers may be used to confirm the presence and identity of endophyte strains artificially inoculated into cultivars and lines.

The endophytes have been genetically characterised to demonstrate genetic distinction from known endophyte strains and to confirm the identity of endophyte strains artificially inoculated into cultivars and lines.

By a 'plurality' of samples of endophytes or plant samples is meant a number sufficient to enable a comparison of genetic and metabolic profiles of individual endophytes. Preferably, between approximately 10 and 1,000,000 endophytes are provided, more preferably between approximately 100 and 1,000 endophytes.

By 'genetic analysis' is meant analysing the nuclear and/or mitochondrial DNA of the endophyte.

This analysis may involve detecting the presence or absence of polymorphic markers, such as simple sequence repeats (SSRs) or mating-type markers. SSRs, also called microsatellites, are based on a 1-7 nucleotide core element, more typically a 1-4 nucleotide core element, that is tandemly repeated. The SSR array is embedded in complex flanking DNA sequences. Microsatellites are thought to arise due to the property of replication slippage, in which the DNA polymerase enzyme pauses and briefly slips in terms of its template, so that short adjacent sequences are repeated. Some sequence motifs are more slip-prone than others, giving rise to variations in the relative numbers of SSR loci based on different motif types. Once duplicated, the SSR array may further expand (or contract) due to further slippage and/or unequal sister chromatid exchange. The total number of SSR sites is high, such that in principle such loci are capable of providing tags for any linked gene.

SSRs are highly polymorphic due to variation in repeat number and are co-dominantly inherited. Their detection is based on the polymerase chain reaction (PCR), requiring only small amounts of DNA and suitable for automation. They are ubiquitous in eukaryotic genomes and have been found to occur every 21 to 65 kb in plant genomes. Consequently, SSRs are ideal markers for a broad range of applications such as genome mapping, trait mapping and marker-assisted selection.

Known SSR markers which may be used to investigate endophyte diversity in perennial ryegrass are described in van Zijll de Jong *et al* (2003).

Alternatively, or in addition, the genetic analysis may involve sequencing genomic and/or mitochondrial DNA and performing sequence comparisons to assess genetic variation between endophytes.

By 'metabolic analysis' is meant analysing metabolites, in particular toxins, produced by the endophytes. Preferably, this is done by preparation of isogenically inoculated plants for each of the endophytes and measurement of toxin levels *in planta.*

By a 'desired genetic and metabolic profile' is meant that the endophyte includes genetic and metabolic characteristics that result in a beneficial phenotype in a plant harbouring, or otherwise associated with, the endophyte.

Such beneficial properties include improved tolerance to water and/or nutrient stress and improved resistance to pests and/or diseases in the plant with which the endophyte is associated.

For example, tolerance to water and/or nutrient stress may be increased by at least approximately 5%, more preferably at least approximately 10%, more preferably at least approximately 25%, more preferably at least approximately 50%, more preferably at least approximately 100%, relative to a control endophyte such as standard toxic (ST) endophyte or to no endophyte control. Preferably, tolerance to water and/or nutrient stress may be increased by between approximately 5% and approximately 50%, more preferably between approximately 10% and approximately 25%, relative to a control endophyte such as ST endophyte or to no endophyte control.

Such beneficial properties also include reduced toxicity of the associated plant to grazing animals.

For example, toxicity may be reduced by at least approximately 5%, more preferably at least approximately 10%, more preferably at least approximately 25%, more preferably at least approximately 50%, more preferably at least approximately 100%, relative to a control endophyte such as ST endophyte. Preferably, toxicity may be reduced by between approximately 5% and approximately 100%, more preferably between approximately 50% and approximately 100% relative to a control endophyte such as ST endophyte.

Preferably, toxicity may be reduced to a negligible amount or substantially zero toxicity.

The methods disclosed herein may be applied to a variety of plants. The methods may be applied to grasses, preferably forage, turf or bioenergy grasses such as those of the genera *Lolium* and *Festuca,* including *L. perenne* (perennial ryegrass) and *L*. *arundinaceum* (tall fescue).

The methods disclosed herein may be applied to a variety of endophytes. The methods may be applied to fungi of the genus *Neotyphodium,* including *N. lolii* and *N*. *coenophialum.* The methods may be applied to fungi of the genus *Epichloë,* including *E*. *festucae* and *E. typhina.* However, the methods may also be used to identify endophytes of previously undescribed taxa.

Applicants have surprisingly found that endophyte E1 is a genetically novel, non-*Neotyphodium lolii,* endophyte. E1 is representative of an as yet un-named taxon. This finding is supported by mitochondrial and nuclear genome sequence analysis.

While applicants do not wish to be restricted by theory, on the basis of DNA specific content, the predicted alkaloid profile of E1 indicates that lolitrem B toxins deleterious to animal health are not produced by this endophyte. Endophyte E1 has the mating-type MAT1-1, the opposite mating-type to that carried by the *N. lolii* endophytes previously characterized. Endophyte E1 also has a high inoculation success rate in perennial ryegrass as compared to other endophytes.

The present invention also provides a substantially purified or isolated endophyte NEA14, which was deposited at the National Measurement Institute on 23 December 2010 with accession number V10/030284.

By 'substantially purified' is meant that the endophyte is free of other organisms. The term therefore includes, for example, an endophyte in axenic culture. Preferably, the endophyte is at least approximately 90% pure, more preferably at least approximately 95% pure, even more preferably at least approximately 98% pure.

The term 'isolated' means that the endophyte is removed from its original environment (eg. the natural environment if it is naturally occurring). For example, a naturally occurring endophyte present in a living plant is not isolated, but the same endophyte separated from some or all of the coexisting materials in the natural system, is isolated.

Disclosed is further identifying and/or cloning nucleic acids including genes encoding polypeptides or transcription factors that are involved in sexual reproduction or vegetative hyphal fusion in an endophyte, for example mating-type genes, such as MAT1-1.

Methods for identifying and/or cloning nucleic acids encoding such genes are known to those skilled in the art and include creating nucleic acid libraries, such as cDNA or genomic libraries, and screening such libraries, for example using probes for genes encoding mating-type genes; or mutating the genome of the endophyte of the present invention, for example using chemical or transposon mutagenesis, identifying changes in the production of polypeptides or transcription factors that are involved in sexual reproduction or vegetative hyphal fusion, and thus identifying genes encoding such polypeptides or transcription factors.

There is provided a substantially purified or isolated nucleic acid encoding a polypeptide or transcription factor that is involved in sexual reproduction or vegetative hyphal fusion in an endophyte.

The nucleic acid may include a mating-type gene, such as MAT1-1, or a functionally active fragment or variant thereof.

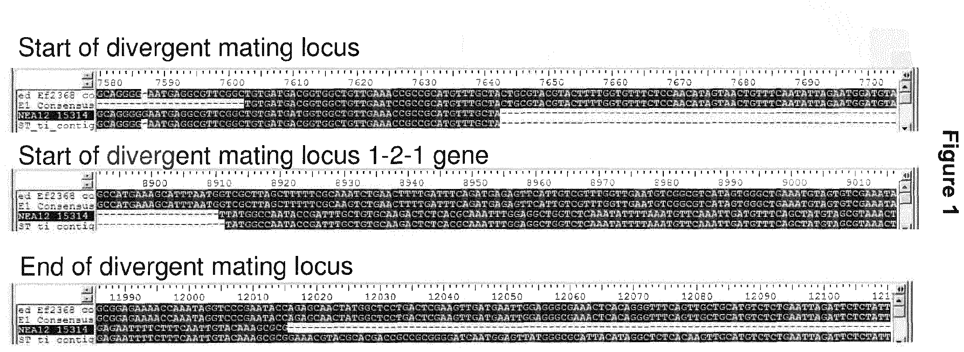

The nucleic acid may include a nucleotide sequence selected from the group consisting of sequences shown in Figure 1 hereto, and functionally active fragments and variants thereof.

By 'nucleic acid' is meant a chain of nucleotides capable of carrying genetic information. The term generally refers to genes or functionally active fragments or variants thereof and or other sequences in the genome of the organism that influence its phenotype. The term 'nucleic acid' includes DNA (such as cDNA or genomic DNA) and RNA (such as mRNA or microRNA) that is single- or double-stranded, optionally containing synthetic, non-natural or altered nucleotide bases, synthetic nucleic acids and combinations thereof.

By a 'nucleic acid encoding a polypeptide or transcription factor involved sexual reproduction or vegetative hyphal fusion' is meant a nucleic acid encoding an enzyme or transcription factor normally present in an endophyte of the present invention, which catalyses or regulates a step involved in sexual reproduction or vegetative hyphal fusion in the endophyte, or otherwise regulates sexual reproduction or vegetative hyphal fusion in the endophyte.

Disclosed are functionally active fragments and variants of the nucleic acids disclosed herein. By 'functionally active' in relation to the nucleic acid is meant that the fragment or variant (such as an analogue, derivative or mutant) is capable of manipulating sexual reproduction or vegetative hyphal fusion in an endophyte, for example by being translated into an enzyme or transcription factor that is able to catalyse or regulate a step involved in sexual reproduction or vegetative hyphal fusion in the endophyte, or otherwise regulate sexual reproduction or vegetative hyphal fusion in the endophyte. Such variants include naturally occurring allelic variants and non-naturally occurring variants. Additions, deletions, substitutions and derivatizations of one or more of the nucleotides are contemplated so long as the modifications do not result in loss of functional activity of the fragment or variant. Preferably the functionally active fragment or variant has at least approximately 80% identity to the relevant part of the above mentioned sequence to which the fragment or variant corresponds, more preferably at least approximately 90% identity, even more preferably at least approximately 95% identity, most preferably at least approximately 98% identity. Such functionally active variants and fragments include, for example, those having conservative nucleic acid changes. Examples of suitable nucleic acid changes are also shown in Figure 1 hereto.

Preferably the fragment has a size of at least 20 nucleotides, more preferably at least 50 nucleotides, more preferably at least 100 nucleotides.

By 'conservative nucleic acid changes' is meant nucleic acid substitutions that result in conservation of the amino acid in the encoded protein, due to the degeneracy of the genetic code. Such functionally active variants and fragments also include, for example, those having nucleic acid changes which result in conservative amino acid substitutions of one or more residues in the corresponding amino acid sequence.

By 'conservative amino acid substitutions' is meant the substitution of an amino acid by another one of the same class, the classes being as follows:
Nonpolar: Ala, Val, Leu, Ile, Pro, Met, Phe, Trp
Uncharged polar: Gly, Ser, Thr, Cys, Tyr, Asn, Gln
Acidic: Asp, Glu
Basic: Lys, Arg, His

Other conservative amino acid substitutions may also be made as follows:
Aromatic: Phe, Tyr, His
Proton Donor: Asn, Gln, Lys, Arg, His, Trp
Proton Acceptor: Glu, Asp, Thr, Ser, Tyr, Asn, Gln

There is provided a genetic construct including a nucleic acid as disclosed herein.

By 'genetic construct' is meant a recombinant nucleic acid molecule.

The genetic construct may be a vector.

By a 'vector' is meant a genetic construct used to transfer genetic material to a target cell.

The vector may be of any suitable type and may be viral or non-viral. The vector may be an expression vector. Such vectors include chromosomal, non-chromosomal and synthetic nucleic acid sequences, eg. derivatives of plant viruses; bacterial plasmids; derivatives of the Ti plasmid from *Agrobacterium tumefaciens;* derivatives of the Ri plasmid from *Agrobacterium rhizogenes;* phage DNA; yeast artificial chromosomes; bacterial artificial chromosomes; binary bacterial artificial chromosomes; vectors derived from combinations of plasmids and phage DNA. However, any other vector may be used as long as it is replicable or integrative or viable in the target cell.

The genetic construct may further include a promoter and a terminator; said promoter, gene and terminator being operatively linked.

By a 'promoter' is meant a nucleic acid sequence sufficient to direct transcription of an operatively linked nucleic acid sequence.

By 'operatively linked' is meant that the nucleic acid(s) and a regulatory sequence, such as a promoter, are linked in such a way as to permit expression of said nucleic acid under appropriate conditions, for example when appropriate molecules such as transcriptional activator proteins are bound to the regulatory sequence. Preferably an operatively linked promoter is upstream of the associated nucleic acid.

By 'upstream' is meant in the 3'->5' direction along the nucleic acid.

The promoter and terminator may be of any suitable type and may be endogenous to the target cell or may be exogenous, provided that they are functional in the target cell.

A variety of terminators which may be employed in the genetic constructs disclosed herein are also well known to those skilled in the art. The terminator may be from the same gene as the promoter sequence or a different gene. Particularly suitable terminators are polyadenylation signals, such as the (CaMV)35S polyA and other terminators from the nopaline synthase (*nos*) and the octopine synthase (*ocs*) genes.

The genetic construct, in addition to the promoter, the gene and the terminator, may include further elements necessary for expression of the nucleic acid, in different combinations, for example vector backbone, origin of replication (ori), multiple cloning sites, spacer sequences, enhancers, introns (such as the maize Ubiquitin *Ubi* intron), antibiotic resistance genes and other selectable marker genes [such as the neomycin phosphotransferase (*nptII*) gene, the hygromycin phosphotransferase (*hph*) gene, the phosphinothricin acetyltransferase (*bar or pat*) gene], and reporter genes [such as beta-glucuronidase (GUS) gene (*gusA*) and the green fluorescent protein (GFP) gene (gfp)]. The genetic construct may also contain a ribosome binding site for translation initiation. The genetic construct may also include appropriate sequences for amplifying expression.

Those skilled in the art will appreciate that the various components of the genetic construct are operably linked, so as to result in expression of said nucleic acid. Techniques for operably linking the components of the genetic construct of the present invention are well known to those skilled in the art. Such techniques include the use of linkers, such as synthetic linkers, for example including one or more restriction enzyme sites.

Preferably, the genetic construct is substantially purified or isolated.

By 'substantially purified' is meant that the genetic construct is free of the genes, which, in the naturally-occurring genome of the organism from which the nucleic acid or promoter of the invention is derived, flank the nucleic acid or promoter. The term therefore includes, for example, a genetic construct which is incorporated into a vector; into an autonomously replicating plasmid or virus; or into the genomic DNA of a prokaryote or eukaryote; or which exists as a separate molecule (eg. a cDNA or a genomic or cDNA fragment produced by PCR or restriction endonuclease digestion) independent of other sequences. It also includes a genetic construct which is part of a hybrid gene encoding additional polypeptide sequence.

Preferably, the substantially purified genetic construct is at least approximately 90% pure, more preferably at least approximately 95% pure, even more preferably at least approximately 98% pure.

The term "isolated" means that the material is removed from its original environment (eg. the natural environment if it is naturally occurring). For example, a naturally occurring nucleic acid present in a living plant is not isolated, but the same nucleic acid separated from some or all of the coexisting materials in the natural system, is isolated. Such nucleic acids could be part of a vector and/or such nucleic acids could be part of a composition, and still be isolated in that such a vector or composition is not part of its natural environment.

As an alternative to use of a selectable marker gene to provide a phenotypic trait for selection of transformed host cells, the presence of the genetic construct in transformed cells may be determined by other techniques well known in the art, such as PCR (polymerase chain reaction), Southern blot hybridisation analysis, histochemical assays (e.g. GUS assays), thin layer chromatography (TLC), northern and western blot hybridisation analyses.

The genetic constructs disclosed herein may be introduced into plants or fungi by any suitable technique. Techniques for incorporating the genetic constructs of the present invention into plant cells or fungal cells (for example by transduction, transfection, transformation or gene targeting) are well known to those skilled in the art. Such techniques include *Agrobacterium*-mediated introduction, Rhizobium-mediated introduction, electroporation to tissues, cells and protoplasts, protoplast fusion, injection into reproductive organs, injection into immature embryos and high velocity projectile introduction to cells, tissues, calli, immature and mature embryos, biolistic transformation, Whiskers transformation, and combinations thereof. The choice of technique will depend largely on the type of plant or fungus to be transformed, and may be readily determined by an appropriately skilled person. For transformation of protoplasts, PEG-mediated transformation is particularly preferred. For transformation of fungi PEG-mediated transformation and electroporation of protoplasts and *Agrobacterium*-mediated introduction of hyphal explants are particularly preferred.

Cells incorporating the genetic constructs disclosed herein may be selected, as described below, and then cultured in an appropriate medium to regenerate transformed plants or fungi, using techniques well known in the art. The culture conditions, such as temperature, pH and the like, will be apparent to the person skilled in the art. The resulting plants may be reproduced, either sexually or asexually, using methods well known in the art, to produce successive generations of transformed plants or fungi.

Disclosed is a plant inoculated with an endophyte as hereinbefore described, said plant comprising an endophyte-free host plant stably infected with said endophyte.

Preferably, the plant is infected with the endophyte by a method selected from the group consisting of inoculation, breeding, crossing, hybridization and combinations thereof.

Preferably, the plant may be infected by isogenic inoculation. This has the advantage that phenotypic effects of endophytes may be assessed in the absence of host-specific genetic effects. More particularly, multiple inoculations of endophytes may be made in plant germplasm, and plantlets regenerated in culture before transfer to soil.

The identification of an endophyte of the opposite mating-type that is highly compatible and stable *in planta* provides a means for molecular breeding of endophytes for perennial ryegrass. Preferably the plant may be infected by hyper-inoculation.

Hyphal fusion between endophyte strains of the opposite mating-type provides a means for delivery of favourable traits into the host plant, preferably via hyper-inoculation. Such strains are preferably selected from the group including an endophyte strain that exhibits the favourable characteristics of high inoculation frequency and high compatibility with a wide range of germplasm, preferably elite perennial ryegrass and/or tall fescue host germplasm and an endophyte that exhibits a low inoculation frequency and low compatibility, but has a highly favourable alkaloid toxin profile.

It has generally been assumed that interactions between endophyte taxa and host grasses will be species specific. Applicants have surprisingly found that endophyte from tall fescue may be used to deliver favourable traits to ryegrasses, such as perennial ryegrass.

Disclosed is further a method of analysing metabolites in a plurality of endophytes, said method including:
providing:
   a plurality of endophytes; and
   a plurality of isogenic plants;
inoculating each isogenic plant with an endophyte;
culturing the endophyte-infected plants; and
analysing the metabolites produced by the endophyte-infected plants.

By 'metabolites' is meant chemical compounds, in particular toxins, produced by the endophyte-infected plant, including, but not limited to, lolines, peramine, ergovaline, lolitrem, and janthitrems, such as janthitrem I, janthitrem G and janthitem F.

By 'isogenic plants' is meant that the plants are genetically identical.

The endophyte-infected plants may be cultured by known techniques. The person skilled in the art can readily determine appropriate culture conditions depending on the plant to be cultured.

The metabolites may be analysed by known techniques such as chromatographic techniques or mass spectrometry, for example LCMS or HPLC. In a particularly preferred embodiment, endophyte-infected plants may be analysed by reverse phase liquid chromatography mass spectrometry (LCMS). This reverse phase method may allow analysis of specific metabolites (including lolines, peramine, ergovaline, lolitrem, and janthitrems, such as janthitrem I, janthitrem G and janthitem F) in one LCMS chromatographic run from a single endophyte-infected plant extract.

LCMS including EIC (extracted ion chromatogram) analysis may allow detection of the alkaloid metabolites from small quantities of endophyte-infected plant material. Metabolite identity may be confirmed by comparison of retention time with that of pure toxins or extracts of endophyte-infected plants with a known toxin profile analysed under substantially the same conditions and/or by comparison of mass fragmentation patterns, for example generated by MS2 analysis in a linear ion trap mass spectrometer.

Disclosed is further a plant, plant seed or other plant part derived from a plant of the present invention and stably infected with an endophyte of the present invention.

Preferably, the plant cell, plant, plant seed or other plant part is a grass, more preferably a forage, turf or bioenergy grass, such as those of the genera *Lolium* and *Festuca,* including *L. perenne* and *L. arundinaceum.*

By 'plant cell' is meant any self-propagating cell bounded by a semi-permeable membrane and containing plastid. Such a cell also required a cell wall if further propagation is desired. Plant cell, as used herein includes, without limitation, seeds suspension cultures, embryos, meristematic regions, callus tissue, leaves, roots, shoots, gametophytes, sporophytes, pollen and microspores.

Disclosed is the use of an endophyte as hereinbefore described to produce a plant stably infected with said endophyte.

Disclosed is a method of quantifying endophyte content of a plant, said method including measuring copies of a target sequence by quantitative PCR.

Preferably, the method may be performed using an electronic device, such as a computer.

Preferably, quantitative PCR may be used to measure endophyte colonisation *in planta,* for example using a nucleic acid dye, such as SYBR Green chemistry, and qPCR-specific primer sets. The primer sets may be directed to a target sequence such as an endophyte gene, for example the peramine biosynthesis *perA* gene.

The development of a high-throughput PCR-based assay to measure endophyte biomass *in planta* may enable efficient screening of large numbers of plants to study endophyte-host plant biomass associations.

As used herein, except where the context requires otherwise, the term "comprise" and variations of the term, such as "comprising", "comprises" and "comprised", are not intended to exclude further additives, components, integers or steps.

Reference to any prior art in the specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

### Detailed description

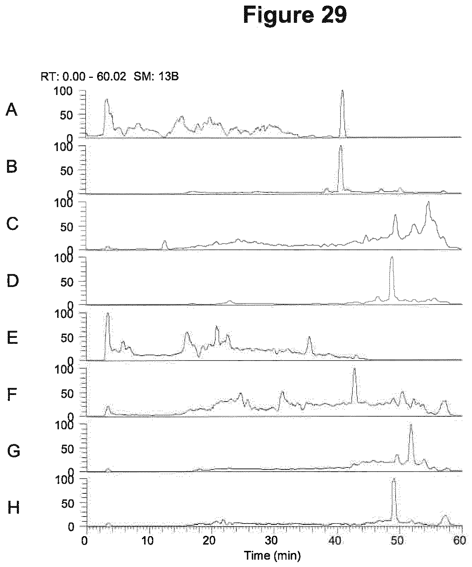

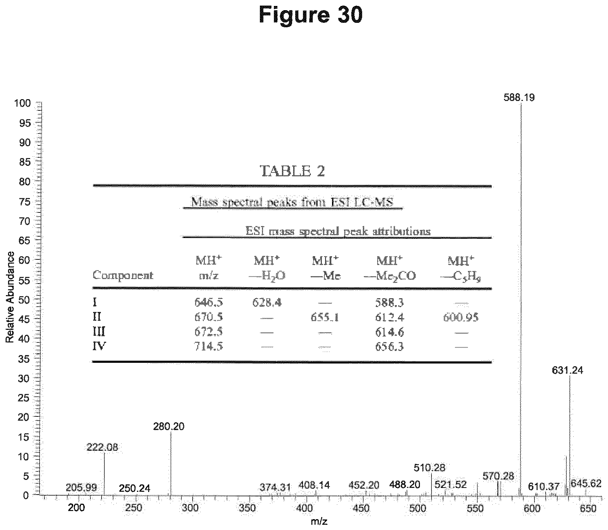

In the figures:
Figure 1 shows sequence alignment analysis of mating-type loci of endophyte strains *E. festucae* strain E2368, E1, NEA12 and ST.

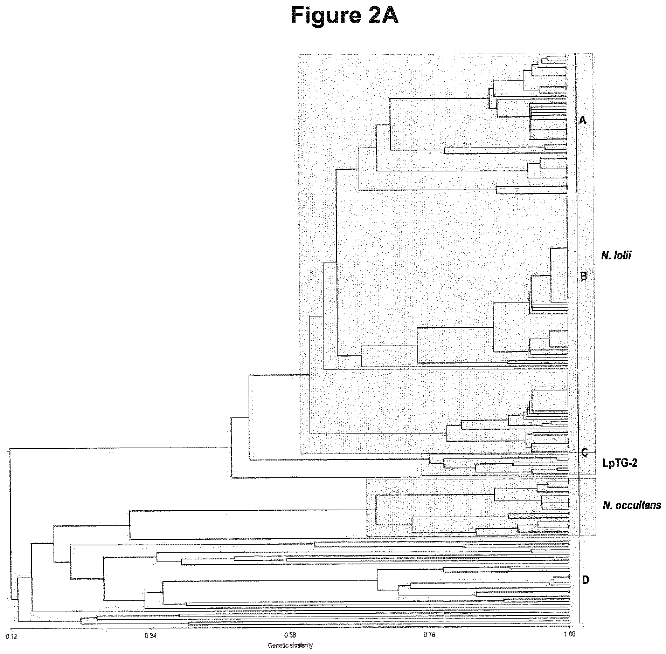

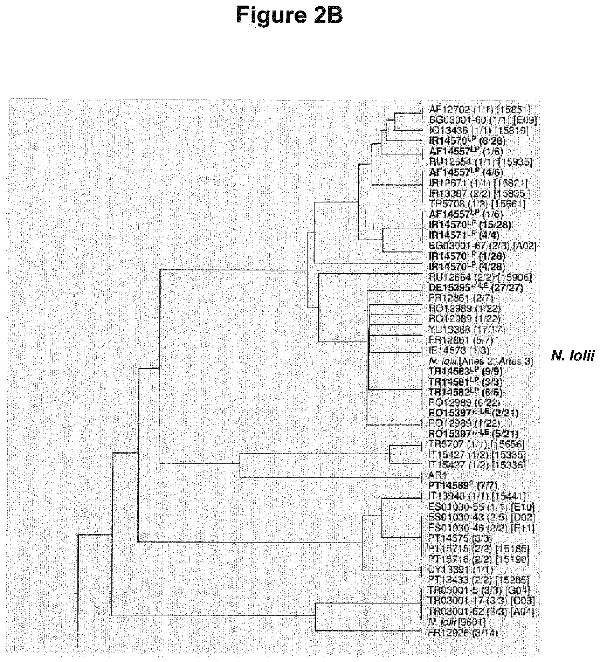

Figure 2A shows a UPGMA phenogram of genetic relationships among endophytes in ryegrass accessions of diverse origins and reference *Neotyphodium* and *Epichloë* species. Genetic identity was measured across 18 SSR loci using the Dice coefficient. Detailed annotations for sections A-D are shown in Figures 2B to 2E, respectively. Specifically, accessions analysed in this study are shaded in grey, the number of genotypes host to that endophyte strain from the total number of genotypes analysed are indicated in the round brackets and a representative host genotype is given in the square brackets. Endophyte isolates from the reference collection are specified in the square brackets following the species name. *N. lolii* Group 1 comprises of isolates Aries 1, Banks 5847, Ellett 5837, Fitzroy 2, Fitzroy 3, KT1-2, North African 6, Vedette 6645 and Victorian 2.
Figure 3 shows isogenic inoculation methodology for endophyte inoculation. A. Meristem callus induction (4 weeks); B. Embryogenic callus proliferation (4 weeks); C. Shoot (and root) regeneration (5 days, 16 hours light); D. Endophyte inoculation; E. Plantlet growth (4 weeks, 16 hours light); F. Growth in soil (3 months); G. SSR-based analysis.
Figure 4 shows the number of hits showing a given percent identity for 250 bp fragments of the NEA12 genome against the *E. festucae* and *N. lolii* genomes. The X-axis shows the percent identity, the Y-axis shows the number of hits. Black: *N. lolii* strain ST; White: *E. festucae* strain E2368.
Figure 5 shows the number of hits showing a given percent identity for 250 bp segments of the E1 genome against the genomes of NEA12, *E. festucae* and *N. lolii.* The X-axis shows the percent identity, the Y-axis shows the number of hits. Black (1st bar in each group): *E. festucae* strain E2368; Grey (2nd bar in each group): Non-*N*. *lolii* strain NEA12; White (3rd bar in each group): *N. lolii* strain ST.
Figure 6 shows the number of hits showing a given percent identity for 250 bp fragments of E1 against NEA12, *E. festucae* and *N. lolii.* The X-axis shows the percent identity, the Y-axis shows the number of hits expressed as a fraction of the total matches seen per comparison. Grey (1st bar in each group): *E. festucae* strain E2368; Black (2nd bar in each group): Non-*N*. *lolii* strain NEA12; White (3rd bar in each group): *N. lolii* strain ST.
Figure 7 shows a schematic diagram of the mating-type loci in *Neotyphodium*/*Epichloë.*
Figure 8 shows ClustalW analysis trees of the sequence flanking the mating-type loci (left), and the NoxR gene (cloned from *E. festucae* strain FL1 gi117413991; right).
Figure 9 shows an alignment between mitochondrial genome of *N. lolii* strain Lp19 and a representative of the Clavicipitaceae, *Metarhizium anisopliae* (Genbank reference number NC_008068.1). While the two mitochondrial genomes vary in size, the genes are present in the same order and strand sense, with differences being due to variable insertions in the *N. lolii* mitochondrial genome.
Figure 10 shows a depiction of part of the block structure of the mitochondrial genomes for each of the fungal endophytes sequenced in this study, as well as *E. festucae* strain E2368 and *Metarhizium anisopliae* for comparison. A shared block (e.g. b84) is present in all 12 mitochondria whereas block 85 is present only in the mitochondria of *E*. *festucae* strain E2368, and Non-*N*. *lolii* strains E1 and NEA12.
Figure 11 shows a mitochondrial genome comparison. Parsimony tree of the relationships between the mitochondrial genomes of the 10 perennial ryegrass endophyte strains sequenced, *E. festucae* strain E2368 and *Metarhizium anisopliae.*
Figure 12 shows a mitochondrial genome comparison. Neighbour joining tree analysis using ClustalW from a DNA alignment of the 40 blocks of sequence (∼ 40kb) that are shared across the 10 perennial ryegrass endophyte strains sequenced, *E. festucae* strain E2368 and *Metarhizium anisopliae.*

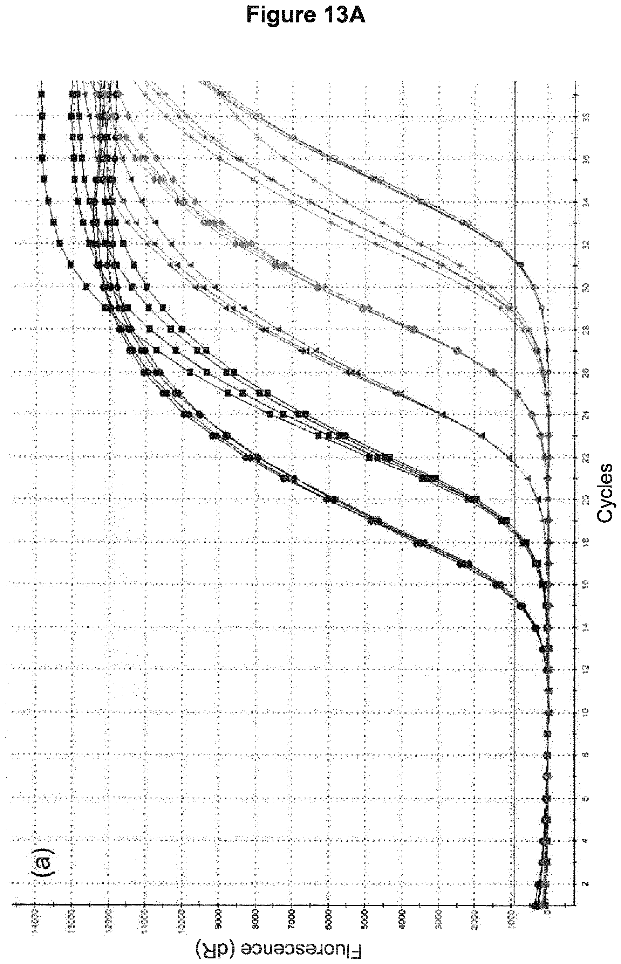

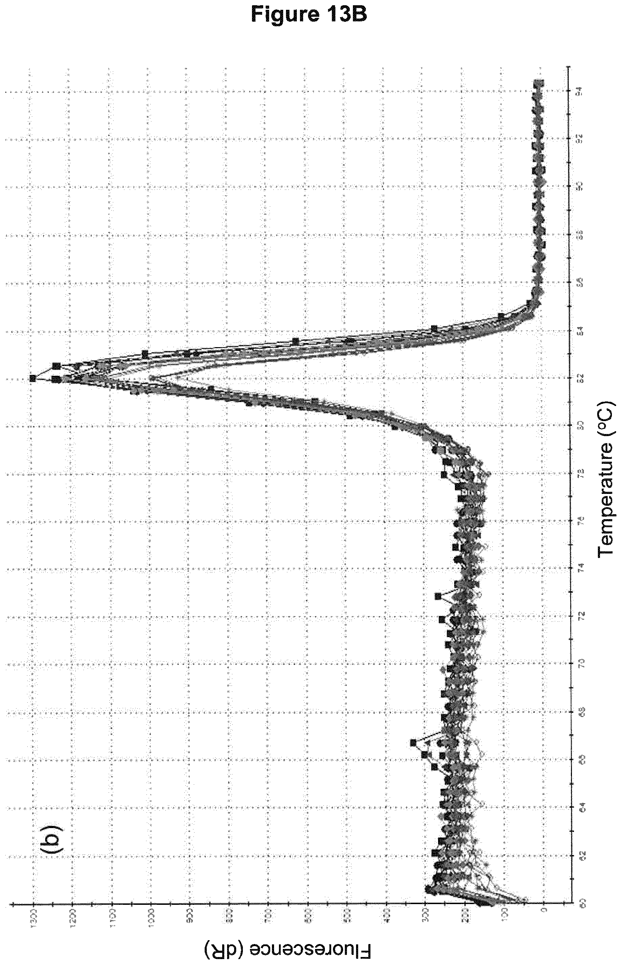

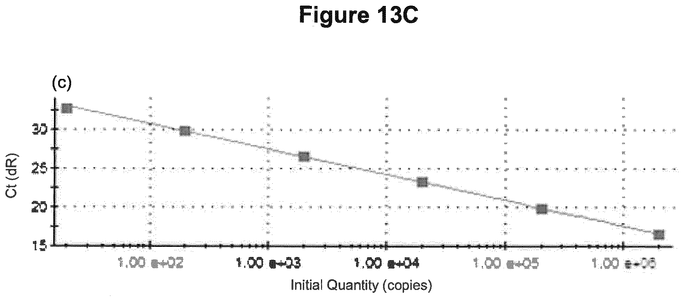

Figure 13 shows a standard curve for quantitative assessment of endophyte colonisation (copy number relative to total plant gDNA). (a) Tight clustering of amplification curves (4 technical replicates) ranging from 2 x 10² to 2 x 10⁶ copies of the 73bp *perA* amplicon. (b) Dissociation curve analysis of the amplification curves shown in (a), with the presence of a single peak indicating primer pair specificity. (c) Assay performance is determined in terms of efficiency, precision and sensitivity. For a typical reaction, a slope of -3.1 to -3.6 and R² value ≥ 0.985 is acceptable. This assay recorded a slope of -3.2 and R² value of 0.999.

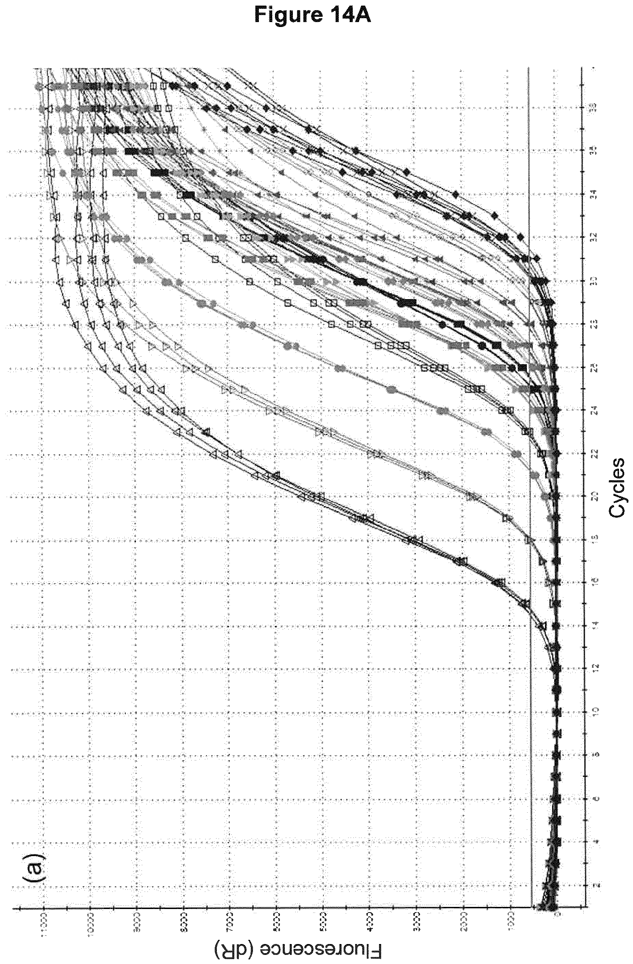

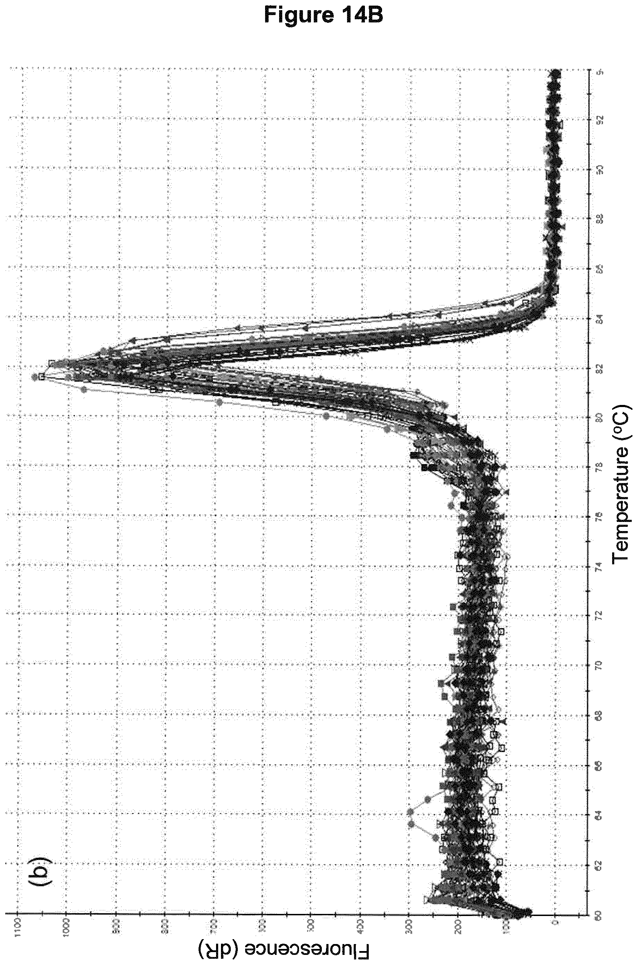

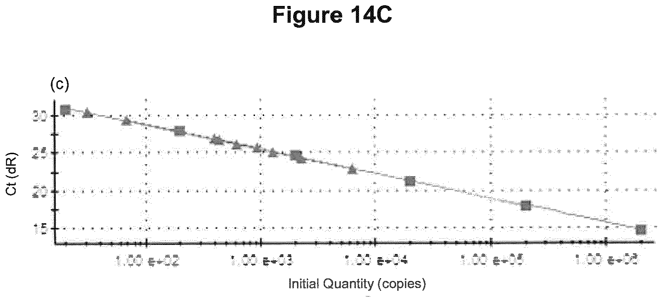

Figure 14 shows a quantitative assessment of endophyte colonisation in diverse ryegrass host panel. (a) Standard curve of *perA* target sequence (2 x 10² to 2 x 10⁶) and amplification curves of the unknown samples. (b) Dissociation curve analysis of the amplification curves shown in (a). (c) Standard curve for *perA* target (■) and unknown samples (▲).
Figure 15 shows a colchicine kill curve of endophyte strain ST mycelia grown in potato dextrose broth at 22°C, 150 rpm for 21 days.
Figure 16 shows phenotype of colchicine treated colonies (0.1 and 0.2%) of endophyte strain ST compared to the untreated ST control. Mycelia were grown on potato dextrose agar at 22°C in dark.
Figure 17 shows an assessment for changes in ploidy level by flow cytometry. a) Dot plots and histogram overlay of control samples, ST, BE9301 and NEA11. b) Dot plots and histogram overlay of two individual ST colonies (13 and 14), showing a shift in peak location relative to the controls.
Figure 18 shows high throughput PCR screening method for detection of lolitrem B gene deletion mutants. The lolitrem genes targeted include: *ItmM* (480 bp), *ItmJ* (734 bp) and *ItmC* (583 bp). M: EasyLadder1 (100-2000 bp); 1-13: Individual putative lolitrem B gene deletion mutants; ST: ST DNA (positive control for *ItmM, ItmJ* and *ItmC*); AR1: AR1 DNA (positive control for *ItmM* and *ItmC,* negative control for *ItmJ*); H₂O PCR control.
Figure 19 shows geographical origins represented in the tall fescue endophyte incidence assessment. This graph shows the 40 different geographic origins represented in the incidence assessment. The X axis gives geographic origins in the alphabetical order and the Y axis shows the number of accessions. The number of negative accessions is shown with black and the number of positive accessions is shown in grey.
Figure 20 shows UPGMA phenogram of genetic relationships among endophytes in tall fescue accessions of diverse origins and reference *Neotyphodium, Epichloë,* FaTG-2 and FaTG-3 species.
Figure 21 shows production of the insecticidal alkaloids loline, loline formate and peramine by tall fescue endophytes in their endogenous host.
Figure 22 shows production of the anti-mammalian alkaloids ergovaline and lolitrem B by tall fescue endophytes in their endogenous host.
Figure 23 shows an example of antifungal bioassay of inhibition reactions. Testing for antifungal activity of endophyte NEA12, ST and AR1 against 8 species of pathogenic fungi.
Figure 24 shows endophytes selected for metabolic profiling in *in vitro* culture. Shown in the top left hand corner is the inhibition score.
Figure 25 shows a method for sampling material for LCMS analysis.
Figure 26 shows a validation assay. *Rhizoctonia cerealis* was grown in the presence of methanol extracts of endophyte mycelia. Shown is an example using the endophyte strain ST. A. Methanol extract of ST grown in the absence of R. *cerealis;* B. Methanol extract ST grown in presence of R. *cerealis;* C. Water only control; D. Methanol only control.
Figure 27 shows structures of endophyte metabolites
   - 1: peramine (MW 247.3);
   - 2: ergovaline (MW 533.6);
   - 3: lolitrem B (MW 685.9);
   - 4: janthitrem I (MW 645.8);
   - 5: janthitrem G (MW 629.8);
   - 6: janthitrem F (MW 645.8).
Figure 28 shows LCMS analysis of standard materials displaying extracted ion chromatogram for the toxins:
   A. peramine
      NL: 7.47E4
      Base Peak m/z = 47.50-248.50 F: ITMS + c ESI Full ms
      [150.00-2000.00] MS
   B. ergovaline
      NL: 1.64E6
      Base Peak m/z = 533.40-534.40 F: ITMS + c ESI Full ms
      [150.00-2000.00] MS
   C. lolitrem B
      NL: 2.25E3
      Base Peak m/z = 685.50-687.00 F: ITMS + c ESI Full ms
      [150.00-2000.00] MS
Figure 29 shows an LCMS comparison of AR37 inoculated perennial ryegrass with NEA12 inoculated perennial ryegrass (IMP04 NEA12 20).
   A. AR37 no peramine
      NL: 3.14E3
      Base Peak m/z = 247.50-248.50 F: ITMS + c ESI
      Full ms [150.00-2000.00] MS
   B. AR37 no ergovaline
      NL: 7.39E4
      Base Peak m/z = 533.40-534.40 F: ITMS + c ESI
      Full ms [150.00-2000.00] MS
   C. AR37 no lolitrem B
      NL: 1.32E4
      Base Peak m/z = 685.50-687.00 F: ITMS + c ESI
      Full ms [150.00-2000.00] MS
   D. AR37 janthitrem
      NL: 8.68E4
      Base Peak m/z = 645.50-646.50 F: ITMS + c ESI
      Full ms [150.00-2000.00] MS
   E. NEA12 no peramine
      NL: 6.18E3
      Base Peak m/z = 247.50-248.50 F: ITMS + c ESI
      Full ms [150.00-2000.00] MS
   F. NEA12 no ergovaline
      NL: 4.10E3
      Base Peak m/z = 533.40-534.40 F: ITMS + c ESI
      Full ms [150.00-2000.00] MS
   G. NEA12 no lolitrem B
      NL: 1.32E4
      Base Peak m/z = 685.50-687.00 F: ITMS + c ESI
      Full ms [150.00-2000.00] MS
   H. NEA12 janthitrem
      NL: 1.04E4
      Base Peak m/z = 645.50-646.50 F: ITMS + c ESI
      Full ms [150.00-2000.00] MS
Figure 30 shows an MSMS analysis of NEA12 insulated perennial ryegrass metabolite 4. Inset is Table 2 from International patent application WO2004/106487 describing the fragmentations of the janthitrems found. Data for NEA12 metabolite 4 is in good agreement with that of component I in the table. (endo15June09-010 #3184 RT: 49.01 AV: 1 NL: 5.02E2, T: ITMS + cESId Full ms2 646.51@cid35.00[165.00-660.00])

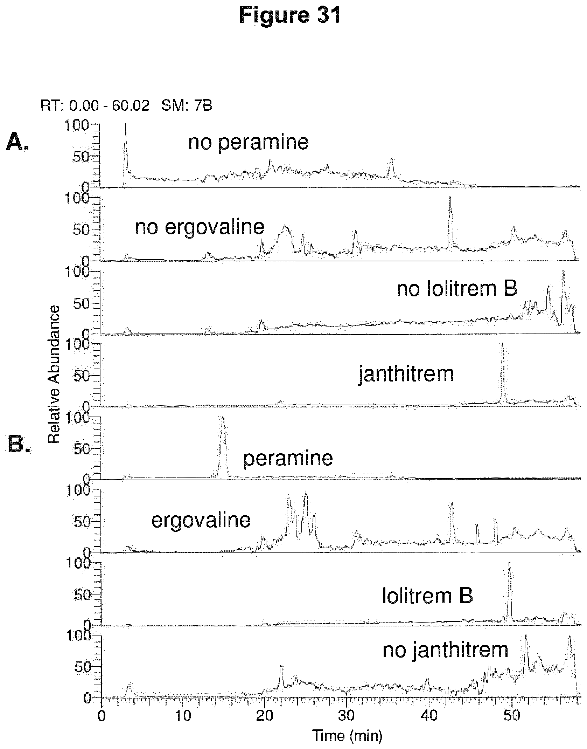

Figure 31 shows Reverse phase liquid chromatography mass spectrometry (LCMS) analysis of A. TOL03 NEA12 and B. TOL03 ST. Profiles show the presence and absence of specific metabolites including peramine, ergovaline, lolitrem, and janthitrems.

### Example 1 - Identification of novel endophytes

Examples not falling within the scope of the claims are for illustrative purposes only.

A collection of 244 perennial grass accessions was assembled for the discovery of novel endophyte strains. The collection targeted accessions from the Northern Mediterranean and Eastern Europe for endophytes that lack lolitrems, as well as accessions from the Middle East, the proposed centre of origin of perennial ryegrass and *N. lolii.*

Genotypic analysis of endophyte content was performed across a total of 189 accessions. From each accession 1-5 plant genotypes were analysed for endophyte. Endophyte incidence was low, with endophyte detected in 51% of accessions. Endophyte was consistently detected (with ≥10 SSR markers) in 77 of the accessions.

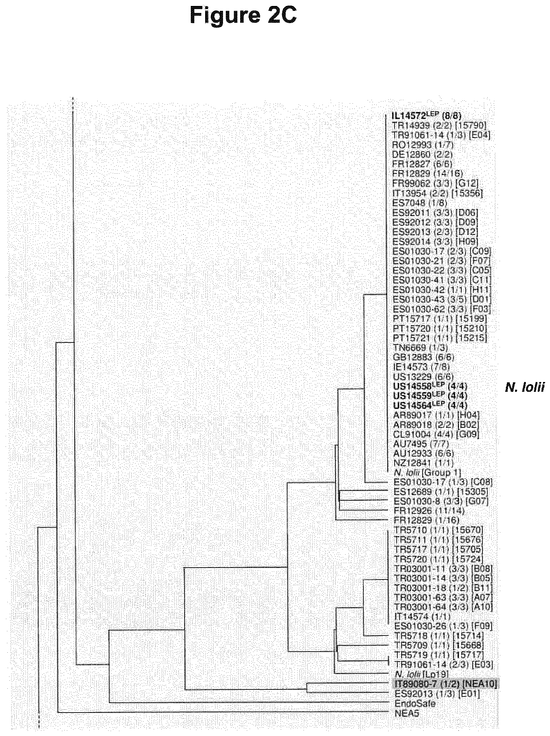

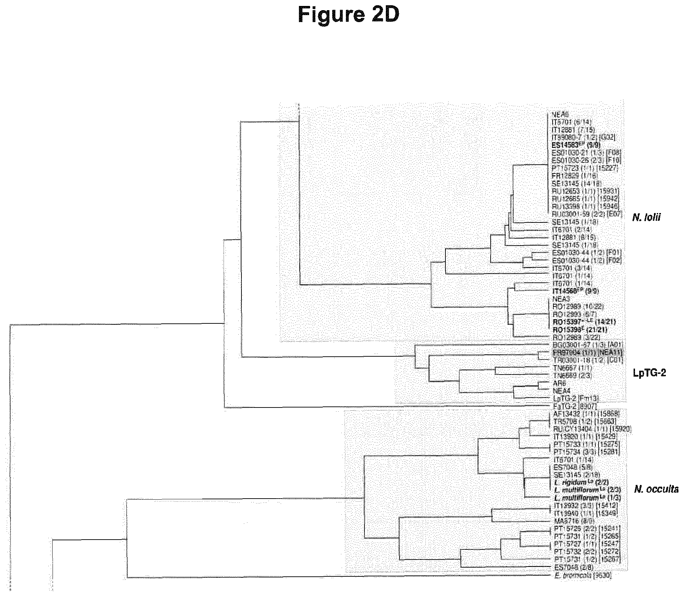

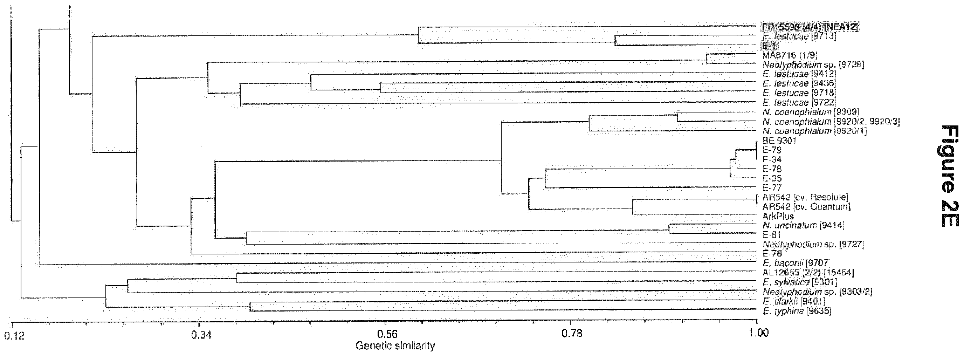

Endophytes representing five different taxa were detected across the 77 accessions with 18 SSR markers used to investigate endophyte diversity in perennial ryegrass (Figure 2). *N. lolii* was predominant, occurring in 63 accessions. Also detected, although less common, were LpTG-2 and putatively new taxa.

Genetic variation in *N. lolii* appeared to be low. A total of 22 unique genotypes were detected across the 63 accessions host to *N. lolii.*

The likely toxin profiles of 14 of the 22 genotypes were established from comparisons with genetic and phenotypic data from previous studies. Most of these genotypes (12/14) showed genetic similarity to endophytes known to produce lolitrems.

There were two genotypes that showed genetic similarity to genotypes known to lack lolitrems but produce ergovaline. One of these genotypes was identical to the genotype detected in the endophyte NEA6. The likely toxin profiles of the remaining eight genotypes were not known. These genotypes did not show high levels of genetic similarity to the endophytes AR1, Endosafe, NEA3 or NEA5.

Plants carrying candidate endophytes were subjected to primary metabolic profiling in the endogenous genetic background, through clonal propagation and measurement of toxin levels. A total of 42 genotypes representing four of the five taxa were selected for toxin profiling, including the eight novel genotypes with unknown toxin profiles. The perennial ryegrass genotype North African 6 (NA₆), which contains standard toxic (ST) endophyte, was used as a control.

For metabolic profiling, a complete randomised block design was used, with four replicate clones for each plant and using four hydroponics tubs as blocks. Following three months in hydroponics, whole shoot (leaf plus basal region) was harvested from each plant. The fresh and dry weights of each plant were measured and powdered sample material from 80 (20 genotypes x 4 replicates) samples (three tillers per sample) analysed for alkaloid content (lolitrem, ergovaline and peramine).

### Example 2 - Candidate endophytes

Candidate endophytes for further study were chosen on the basis of their genetic identity and metabolic profile. Host-endophyte combinations producing significant amounts of lolitrem B were eliminated, as the ryegrass staggers syndrome produced by this alkaloid is the most important limitation for livestock production.

The candidate endophyte NEA10 (originating from Spain) was identified as a novel genotype in this analysis with an unknown toxin profile. Its genetic identity is a unique *N. lolii* strain. Following *in planta* metabolic profiling analysis, candidate endophyte NEA10 was found to produce ergovaline and peramine, and not lolitrem B.

The candidate endophyte NEA11 (originating from France) was identified as a novel genotype in this analysis with an unknown toxin profile. Its genetic identity is a unique LpTG-2 strain. Following *in planta* metabolic profiling analysis, candidate endophyte NEA11 was found to produce ergovaline and peramine, and not lolitrem B.

The candidate endophyte NEA12 (originating from France) was identified as a novel genotype in this analysis with an unknown toxin profile. NEA12 is a genetically novel, non-*Neotyphodium lolii,* endophyte representative of an as yet un-named taxon. Following *in planta* metabolic profiling analysis, candidate endophyte NEA12 was found to not produce the three alkaloids assessed (lolitrem B, ergovaline and peramine).

The candidate endophyte E1 was identified as a novel genotype in this analysis with an unknown toxin profile. E1 is a genetically novel, non-*Neotyphodium lolii,* endophyte representative of an as yet un-named taxon. Following *in planta* metabolic profiling analysis, candidate endophyte E1 was found to not produce the three alkaloids assessed (lolitrem B, ergovaline and peramine).

### Example 3 - Methodologies for endophyte characterisation

### Endophyte isolation

Novel candidate endophytes were isolated from their host plant to establish an *in vitro* culture. Following isolation, the genotype of each endophyte was confirmed by SSR analysis to ensure a high level of quality control prior to inception of isogenic inoculations.

### Establishment of meristem cultures for a diverse perennial ryegrass host panel

A set of cultivars representing elite germplasm were obtained, including forage and turf types. Meristem cultures from different cultivars were established to evaluate and compare the phenotypic properties of novel endophyte strains in diverse isogenic host backgrounds. Embryogenic genotypes were identified for each of the cultivars through callus induction and proliferation. Subsequent regeneration of embryogenic genotypes identified primary tissue culture responsive (pTCR) genotypes for each of the cultivars. The number of pTCR genotypes with regeneration frequencies ranging from 80-100% varied from 1-4 per cultivar. pTCR genotypes were then prepared for meristem-derived callus induction to identify highly regenerable genotypes for isogeneic endophyte inoculation. Table 1 shows a selection of cultivars developed, and the tissue culture responsive (TCR) genotype, used for isogenic inoculation.

**Table 1. Summary information for perennial cultivars selected for isogenic inoculation.**

| ***Cultivar*** | ***Characteristics*** | ***TCR genotype used for inoculation*** |
|---|---|---|
| Bealey | Tetraploid forage type | Bea 02 |
| Bronsyn | Standard forage type with robust endophyte performance | Bro 08 |
| Impact | Late flowering, dense tillering | Imp 04 |
| Barsandra Tolosa | forage type Turf type Distinct forage type | San 02 Tol 03 |

### Isogenic inoculation of novel perennial ryegrass endophytes

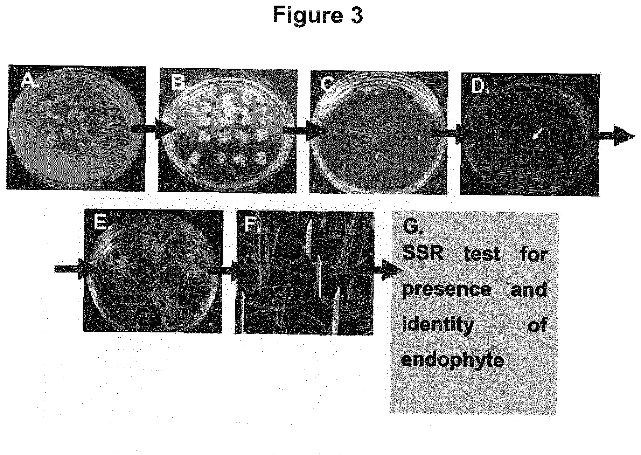

In order to accurately determine the phenotypic effects of different candidate endophytes in the absence of host-specific genetic effects, a system for isogenic inoculation was developed (Figure 3). The regenerating callus method of inoculation was chosen, as it results in a relatively high rate of inoculation compared to other tested techniques, and the achieved isogenic inoculation rate was similar to the standard inoculation procedure for non-isogenic seedlings. Novel candidate endophytes NEA10, NEA11, NEA12, E1 and control endophyte ST were individually inoculated into elite germplasm. The logistical approach was to inoculate two cultivars at any given time, with one TCR genotype for each variety chosen for inoculation in this initial study. For each cultivar-endophyte combination, 30 replicate inoculations were performed, 25 of these replicates being transferred to soil. Following inoculation and plantlet regeneration in culture, plants were transferred to soil for three months to allow establishment of endophyte and host-plant associations. After this period, three tillers from each plant were sampled and tested for endophyte presence using SSR-based analysis.

A quantitative score was used to assess endophyte inoculation frequency (Table 2). Three diagnostic SSR markers were used to determine endophyte presence and identity and samples were scored on a scale of 0-3.

Of the 570 inoculations tested, 195 (34.2%) could be positively scored with a high degree of confidence (Table 3). Successful inoculations are listed on Table 3.

**Table 2. SSR screening for endophyte presence in planta.**

| ***Quantitative score*** | ***Alleles present and of correct size for given SSR loci*** |
|---|---|
| 3 | Endophyte present |
| 2 | Endophyte present |
| 1 | Endophyte absent |
| 0 | Endophyte absent |

**Table 3. Summary statistics for isogenic inoculation of selected candidate endophytes into a targeted perennial ryegrass panel of 5 hosts.**

| **A. Number of positive inoculants** | | | | | | |
|---|---|---|---|---|---|---|
| | ***NEA10*** | ***NEA11*** | ***NEA12*** | ***E1*** | ***ST*** | ***Total*** |
| **Bea02** | 0 | 12 | 3 | 4 | 8 | 27 |
| **Bro08** | 0 | 14 | 1 | 13 | 13 | 41 |
| **Imp04** | 3 | 40 | 4 | 10 | 16 | 73 |
| **San02** | 0 | 17 | 6 | 6 | 11 | 40 |
| **Tol03** | 0 | 3 | 2 | 6 | 3 | 14 |
| **Total** | 3 | 86 | 16 | 39 | 51 | **195** |

| **B. Total number of inoculations tested** | | | | | | |
|---|---|---|---|---|---|---|
| | ***NEA10*** | ***NEA11*** | ***NEA12*** | ***E1*** | ***ST*** | ***Total*** |
| **Bea02** | 24 | 18 | 20 | 19 | 25 | 106 |
| **Bro08** | 19 | 15 | 20 | 18 | 25 | 97 |
| **Imp04** | 31 | 49 | 21 | 12 | 35 | 148 |
| **San02** | 47 | 39 | 24 | 7 | 32 | 149 |
| **Tol03** | 17 | 7 | 18 | 17 | 11 | 70 |
| **Total** | 138 | 128 | 103 | 73 | 128 | **570** |

| **C. Percent of positive inoculants** | | | | | | |
|---|---|---|---|---|---|---|
| | ***NEA10*** | ***NEA11*** | ***NEA12*** | ***E1*** | ***ST*** | ***Average*** |
| **Bea02** | 0.0 | 66.7 | 15.0 | 21.1 | 32.0 | 25.5 |
| **Bro08** | 0.0 | 93.3 | 5.0 | 72.2 | 52.0 | 42.3 |
| **Imp04** | 9.7 | 81.6 | 19.0 | 83.3 | 45.7 | 49.3 |
| **San02** | 0.0 | 43.6 | 25.0 | 85.7 | 34.4 | 26.8 |
| **Tol03** | 0.0 | 42.9 | 11.1 | 35.3 | 27.3 | 20.0 |
| **Average** | 2.2 | 67.2 | 15.5 | 53.4 | 39.8 | **34.2** |

Variation in inoculation success according to candidate endophyte identity was observed (Table 3). Endophyte NEA10 (2.2%), for example, exhibited relatively lower success rates as compared to NEA11 (67.2%), or the commercial endophyte ST (39.8%; Table 4) and only formed stable associations with one of the five hosts in the panel (Impact). Endophyte E1 is a highly compatible endophyte, which obtained a high rate of success of inoculation into perennial ryegrass (Table 3) compared to other endophytes examined, including the strain ST.

Variation was also observed between host plant genotypes for successful inoculations (Table 3). Tolosa (20.0%) appears to be more recalcitrant to inoculation compared to host plants such as Bronsyn (42.3%) and Impact (49.3%).

### Vegetative stability of isogenic perennial ryegrass-fungal endophyte associations

Fully confirmed endophyte positive plants from the targeted host-endophyte panel (host plants Bealey, Bronsyn, Barsandra, Tolosa and Impact; endophytes ST, NEA10, NEA11, NEA12) were retested 6-12 months after inoculation and 18-24 months after inoculation, to confirm the presence of endophyte and to assess vegetative stability. In this experiment, 3 replicates of 3 tillers each (total of 9 tillers) were collected for SSR-based analysis.

Most of the previously confirmed endophyte positive plants were again confirmed in this study at 6-12 months post inoculation, indicating that each of the host - endophyte combinations were stable (Table 4). Endophyte NEA12 appears to be less stable *in planta,* as 7 of the 13 previously confirmed samples could not be fully confirmed in this experiment (Table 4). ST also showed lower levels of stability compared to NEA11, with 7/21 samples not re-confirmed in this study (Table 4). Following this analysis, up to three independent inoculation events from each host plant - endophyte combination were retained for further study.

At 18-24 months post inoculation, plants were further assessed for long term vegetative stability (Table 4). ST, NEA10 and NEA11 each exhibit stable associations, with most plants retaining endophyte. NEA12 appears to be less stable in some associations, however does form stable long term associations with Tolosa.

**Table 4. Endophyte frequency in priority ryegrass host panel genotypes in re-sampled plants that were previously fully confirmed. Plants were re-sampled 6-12 months (shown in bold text) post inoculation and again after 18-24 months post inoculation (shown in normal text). NA = not applicable, as no fully confirmed plants were previously identified.**

| **Plant genotype** | **Endophyte genotype** | | | |
|---|---|---|---|---|
| | **ST** | **NEA10** | **NEA11** | **NEA12** |
| **Impact** | **9/10** | **2/3** | **12/12** | **1/4** |
| **(Imp04)** | 3/3 | 2/2 | 3/3 | 1/1 |
| **Barsandra** | **4/6** | | **7/7** | **2/4** |
| **(San02)** | 2/3 | **NA** | 3/3 | 1/2 |
| **Tolosa** | **1/2** | | **3/3** | **2/2** |
| **(Tol03)** | 1/1 | **NA** | 2/3 | 2/2 |
| **Bealey** | **3/3** | | **9/9** | **0/2** |
| **(Bea02)** | 2/3 | **NA** | 3/3 | 0/1 |
| **Bronsyn** | **3/6** | | **9/9** | **1/1** |
| **(Bro08)** | 2/2 | **NA** | 4/4 | 0/1 |

### Metabolic profiling of isogenic perennial ryegrass-fungal endophyte associations

Metabolic profiling was conducted to determine the stability of the predicted endophyte phenotype in a range of different host genotype backgrounds. Four replicates of three tillers each were grown under optimal conditions in hydroponics for six weeks prior to measuring lolitrem B, ergovaline and peramine levels. Each replicate plant was also tested for the presence/identity of endophyte using SSR-based genotyping in order to correlate toxin profile with endophyte presence, in particular for those instances were toxin profiles were negative for the alkaloids measured.

Table 5 summarises the outcomes of metabolic profiling in hydroponics for both the endophyte discovery phase and the isogenic inoculation phase. Toxin profiles were as predicted from the cluster assignment of the endophyte in the diversity analysis and the toxin profiles measured in the endogenous host plant.

**Table 5. Metabolic profile of candidate endophytes.**

| ***Endophyte strain*** | ***Endogenous toxin profile*** | ***Isogenic toxin profile^{b}*** | ***Origin*** | ***Species*** |
|---|---|---|---|---|
| NEA10 | -/E/n.d^{a}/- | -/E/P/- | Spain | *N. lolii* |
| NEA11 | -/E/n.d/- | -/E/P/- | France | *Lp*TG-2 |
| NEA12 | -/-/-/n.d | -/-/-/J | France | non-*N*. *lolii* |
| E1 | n.d | -/-/-/- | | non-*N*. *lolii* |
| ST | L/E/P/- | L/E/P/- | | *N. lolii* |

| | | | | |
|---|---|---|---|---|
| Toxins are listed in order: L = Lolitrem B; E = Ergovaline; P = Peramine; J = Janthitrems ^{a} Peramine not measured in NEA10 and NEA11 samples; Janthitrems not measured in NEA12 samples ^{b} Toxin profile in isogenic associations | | | | |

### Genome Survey Sequencing of candidate fungal endophytes

### Nuclear genome assembly

Genome Survey Sequencing was performed for non-*N*. *lolii* strains NEA12 and E1, LpTG-2 strain NEA11 and *Neotyphodium lolii* strains including Standard Toxic (ST) and NEA10 using GSFLX Titanium (TI-GSFLX) pyrosequencing technology (Roche; as per manufacturers instructions). A further five *N. lolii* strains were sequenced using either GSFLX Standard or GS20 pyrosequencing technology. Genome assembly for each of the strains was conducted with GSFLX De Novo Assembler (Table 6).

A new genome assembly was performed for *N. lolii* strain ST (GSFLX De Novo Assembler), combining sequence reads from both GSFLX and TI-GSFLX runs. Table 7 compares the assembly of single and multiple strains. This combined assembly of the ST genome achieves c.12x coverage of the c.32 Mbp haploid genome. The genome is assembled into 7,875 large contigs (0.5 to 47 kb) of which the net length is 31,750,111 bp.

Analysis using Augustus gene prediction software trained for *Fusarium graminearum* shows that there are 11,517 predicted protein coding genes in the *N. lolii* genome.

**Table 6. Summary statistics for GS-FLX based whole genome sequencing of candidate endophytes.**

| | ***N.lolii* Lp19** | ***N.lolii* ST** | ***N. lolii* NEA3** | ***N. lolii* AR1** | ***N. lolii* E9** | ***N.lolii* G4** | ***N.lolii* NEA10** | ***N.lolii* ST** | ***non-N.lolii* E1** | **non-*N.lolii* NEA12** | **LpTG-2 NEA11** |
|---|---|---|---|---|---|---|---|---|---|---|---|
| Genome size (Mb) | ∼29 | ∼29 | ∼29 | ∼29 | ∼29 | ∼29 | ∼29 | ∼28 | TBD | TBD | ∼55 |
| Toxin profile^{a} | L+E+P | L+E+P | E+P | P | L+P | L+P | E+P | L+E+P | TBD | J | E+P |
| 454 Sequencer | GS20 | GSFLX Standard | GSFLX Standard | GSFLX Standard | GSFLX Standard | GSFLX Standard | GSFLX Titanium | GSFLX Titanium | GSFLX Titanium | GSFLX Titanium | GSFLX Titanium |
| Number of sequencing runs | 1 | 1 | 1 | 1 | 1 | 1 1/2 | 1/2 | 1 | 1/2 | 1/2 | 1/2 |
| Number of high quality reads | 449,408 | 288,527 | 361,154 | 437,465 | 344,074 | 631,248 | 580,060 | 1,220,036 | 539,019 | 399,868 | 456,111 |
| Number of bases in high quality reads | 47,820,858 | 71,810,513 | 84,032,924 | 97,510,674 | 85,419,382 | 146,574,403 | 221,859,987 | 451,459,919 | 202,854,865 | 165,826,144 | 177,307,015 |
| Average read length (bases) | 106 | 249 | 232 | 223 | 249 | 215 | 383 | 370 | 377 | 415 | 389 |
| Origin of reads assembled^{b} | nuclear + mt | nuclear + mt | nuclear + mt | nuclear + mt | nuclear + mt | nuclear + mt | nuclear + mt | nuclear | nuclear + mt | nuclear + mt | nuclear + mt |

| Large contigs (>500 bases) | | | | | | | | | | | |
|---|---|---|---|---|---|---|---|---|---|---|---|
| Number of contigs | 6 | 2,524 | 5,251 | 6,070 | 6,612 | 12,663 | 7,272 | 4,198 | 9,139 | 12,399 | 14,791 |
| number of bases | 99,508 | 1,834,624 | 3,911,733 | 4,650,113 | 5,208,116 | 12,393,467 | 26,931,240 | 24,382,151 | 27,150,736 | 17,300,350 | 16,306,033 |
| average contig size | 16,584 | 726 | 744 | 766 | 787 | 978 | 3703 | 5,808 | 2970 | 1,395 | 1,102 |
| N50 contig size | 88,709 | 680 | 723 | 751 | 774 | 1039 | 7668 | 11,026 | 5845 | 1,703 | 1,214 |
| largest contig size (bases) | 88,709 | 65,108 | 15,473 | 19,024 | 81,839 | 29,071 | 50,291 | 90,675 | 40,456 | 16,319 | 59,986 |

| All contigs | | | | | | | | | | | |
|---|---|---|---|---|---|---|---|---|---|---|---|
| number of contigs | 29,013 | 28,137 | 33,262 | 33,777 | 33,136 | 32,796 | 11,809 | 6,962 | 15,589 | 20,640 | 39,791 |
| number of bases | 3,532,954 | 7,999,326 | 10,842,510 | 11,755,707 | 12,022,601 | 17,790,671 | 28,155,780 | 25,104,969 | 28,916,589 | 19,862,340 | 23,307,237 |

| | | | | | | | | | | | |
|---|---|---|---|---|---|---|---|---|---|---|---|
| ^{a}L= Lolitrem B, E= Ergovaline, P= Peramine, J= Janthitrems ^{b}Newbler Assembler | | | | | | | | | | | |

**Table 7. Assembly comparison of single and multiple strains of N. lolii endophyte**

| | **Lp19** | **ST** | **NEA3** | **AR1** | **E9** | **G4** | **ST** | **combined ST** | **ST+NEA3+AR1+E9+G4** |
|---|---|---|---|---|---|---|---|---|---|
| 454 Sequencer | GS 20 | GS FLX (Standard) | GS FLX (Standard) | GS FLX (Standard) | GS FLX (Standard) | GS FLX (Standard) | GS FLX (Titanium) | GS FLX (Standard + Titanium) | GS FLX (Standard) |
| Number of sequencing runs | 1 | 1^{a} | 1 | 1 | 1^{a} | 1^{b} | 1 | 2 | 5 |
| Numberof reads | 282,604 | 191,848 | 257,381 | 311,444 | 267,445 | 446,017 | 913,566 | 1,105,414 | 1,474,135 |
| Number of bases in reads | 28,628,965 | 46,613,713 | 58,666,512 | 68,947,121 | 65,192,155 | 101,770,051 | 334,946,727 | 381,560,440 | 341,189,552 |

| Large contigs (≥ 500 bases) | | | | | | | | | |
|---|---|---|---|---|---|---|---|---|---|
| number of contigs | 109 | 1,419 | 3,210 | 3,519 | 4,560 | 11,895 | 8,825 | 7,875 | 11,905 |
| number of bases | 124,393 | 909,187 | 2,111,227 | 2,317,893 | 3,041,084 | 9,249,140 | 31,669,111 | 31,750,111 | 26,515,831 |
| average contig size | 1,141 | 640 | 657 | 658 | 666 | 777 | 3,588 | 4,031 | 2,227 |
| N50 contig size^{c} | 1,193 | 606 | 632 | 636 | 644 | 774 | 6,142 | 7,231 | 3,436 |
| largest contig size (bases) | 7,867 | 8,639 | 7,382 | 8,816 | 8,016 | 8,226 | 46,664 | 46,668 | 24,527 |
| Q40 plus bases (%)^{d} | 90.62 | 93.97 | 93.43 | 93.90 | 93.72 | 94.64 | 98.03 | 98.52 | 98.32 |

| All contigs (≥ 100 bases) | | | | | | | | | |
|---|---|---|---|---|---|---|---|---|---|
| number of contigs | 2,183 | 8,769 | 12,434 | 15,324 | 15,126 | 28,456 | 11,324 | 10,555 | 21,836 |
| number of bases | 500,187 | 2,911,985 | 4,778,407 | 5,584,622 | 6,123,678 | 14,307,808 | 32,350,805 | 32,482,543 | 29,165,397 |

### Alkaloid biosynthetic gene content

The content of genes known to be involved in alkaloid production in each of the sequenced endophyte genomes was investigated. Sequence reads for each of the strains were subjected to a BLAST(N) search against each of the known toxin gene sequences (downloaded from NCBI) to determine the degree of gene coverage by sequence reads. Table 8 below shows the correlation between secondary metabolite production and toxin-related gene content in endophyte genomes.

Based on this analysis, endophyte strain E1 is predicted to produce the alkaloids peramine and ergovaline, but not loline or lolitrem B. *In planta* analysis of alkaloid content has shown that E1 does indeed not produce loline or lolitrem B.

NEA10 and NEA11 produce ergovaline and peramine, but not lolitrem B. The NEA11 sequence provides evidence for 2 peramine biosynthesis genes, as might be expected in a heteroploid genome.

NEA12, known to lack production of ergot alkaloids and lolitrem B, also lacks corresponding biosynthetic genes.

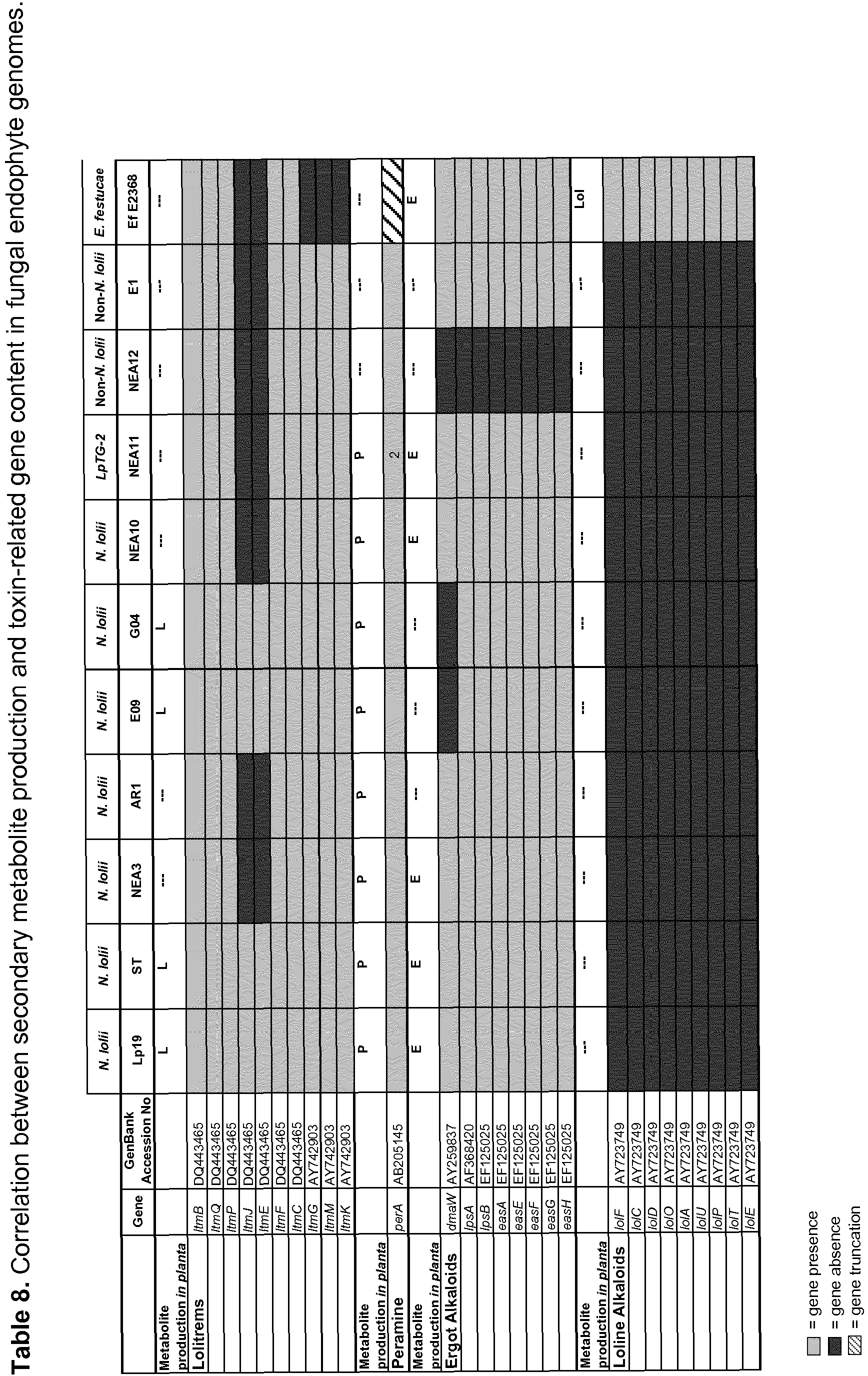

### Nuclear Genome Comparison

### Comparison of the NEA12 nuclear genome to E. festucae E2368 and N. lolii ST

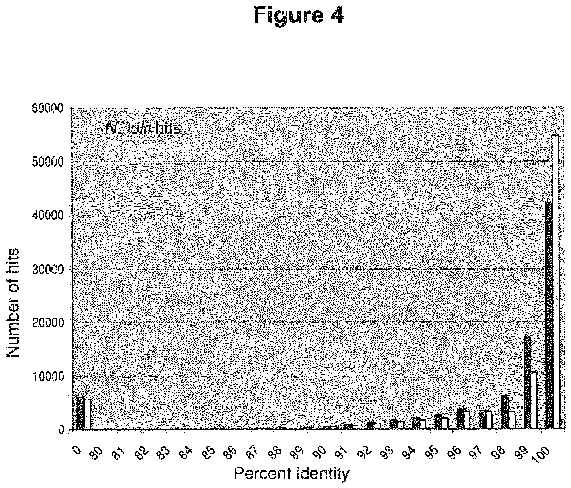

To compare the nuclear genome of NEA12 to *E. festucae* and *N. lolii,* the contigs derived from NEA12 were split into 250 bp segments and these segments were used as BLAST(N) queries against *E. festucae* strain E2368 (University of Kentucky, http://www.genome.ou.edu.fungi.html) and *N. lolii* ST contigs. One hit was scored for each 250 bp contig if it was greater than 50 bp long and greater than 80% identity. Summary statistics were taken for NEA12 250bp fragments against *E. festucae* and N. *lolii* (Figure 4).

The number of hits showing a given percent identity shows there are more 250 bp segments that give 100 percent identity matches against an *E. festucae* genome than a *N. lolii* genome.

The above statistic is independent of the length of the overlap. An identical 250 bp region would give a 250 bp overlap with a percent identity of 100. The number and proportion of these identical reads is given for the two searches below (Table 9).

**Table 9. The number and proportion of identical reads between NEA12 and an E. festucae genome and a N. lolii genome.**

| | **ST** | ***E. festucae*** | **Total** |
|---|---|---|---|
| Number of identical reads (100% identity between 250 bp segment) | 16914 | 28866 | 89416 |
| Percent of identical reads (100% identity between 250 bp segment) | 18.92 | 32.28 | |

There are also segments that have no match to either *N. lolii* (6051) or *E. festucae* (5670). These data suggest that NEA12 is a new endophyte taxon that is genetically closer to *E. festucae* than *N. lolii.* This data supports the earlier observation, using SSR-based genetic diversity analysis, that NEA12 is genetically distinct from *N. lolii* and *E. festucae.*

### Comparison of E1 nuclear genome to NEA12, E. festucae E2368 and N. lolii ST

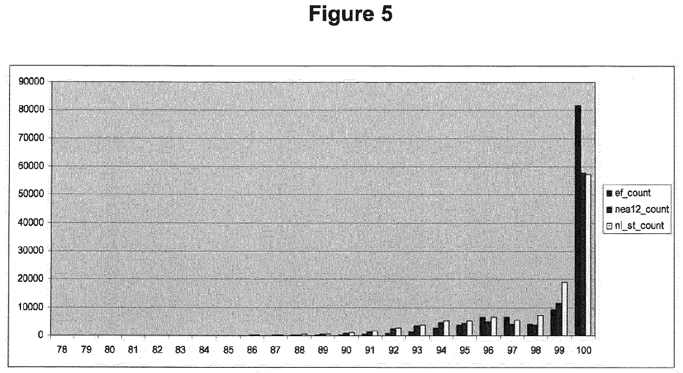

For comparison at the whole genome level, the contigs from endophyte strain E1 were split into 123,258 250 bp segments. Each 250 bp segment was used as a BLAST(N) query against the assembled whole genome DNA sequences from NEA12, *E. festucae* E2368 and *N. lolii* ST (Figure 5). A BLAST(N) hit was recorded if there was an overlap of greater than 49 bp. The number of overlaps at a given percent identity was counted for each search. The plot of this data reveals that the genome of endophyte strain E1 is more similar to that of *E. festucae* strain E2368 than to either *N. lolii* strain ST or NEA12.

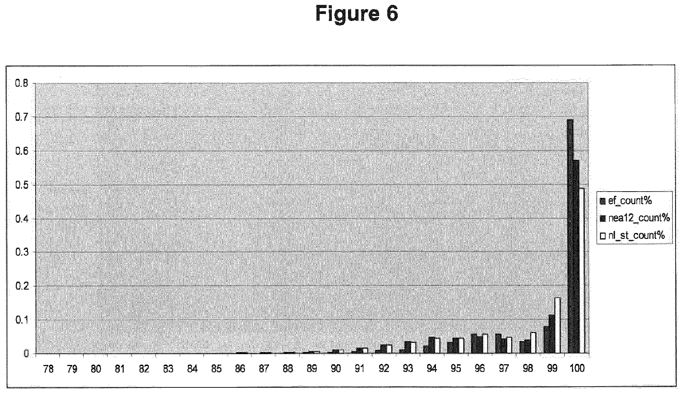

The assembled contigs from NEA12 sum to c.17.3 Mb, so the level of sequence similarity to that endophyte is probably underestimated due to limited scope for comparison. If the similarity is expressed as a fraction of the total matches observed per comparison, strain E1 is seen to be more similar to strain NEA12 than to *N. lolii* strain ST (Figure 6). The property of enhance similarity between E1 and *E. festucae* as compared to *N. lolii* is similar to the pattern seen with mitochondrial genome analysis.

### LpTG-2 endophyte NEA11

The LpTG-2 endophyte strain NEA11 is reported to be a hybrid of *N. lolii* and *E. typhina.*

Mitochondrial sequence analysis supports the hybridisation of *E. typhina* with a *N. lolii* with only the *N. lolii* mitochondria being retained.

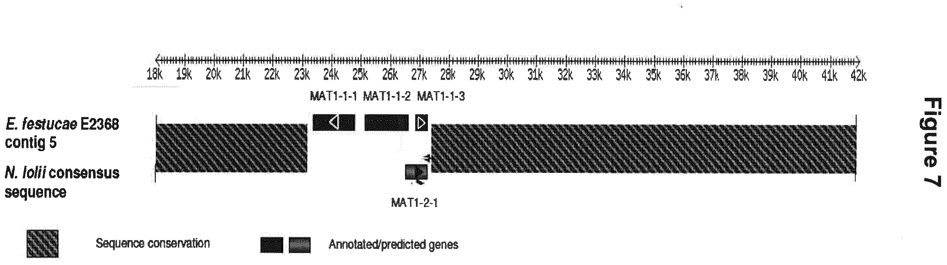

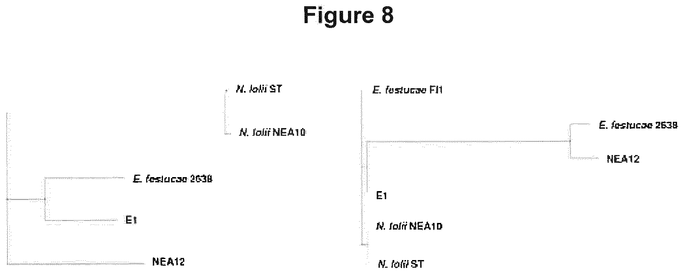

Evidence for the hybrid nuclear genome is seen when nuclear genes are used as a query against contigs from the NEA11 genome assembly (Figures 7 and 8).

The panels below show a region of the 'UDP-N-acetylglucosaminyltransferase' gene from *E. festucae* being used as a BLAST(N) query against: *E. festucae* (E2368) genome contigs; *N. lolii* (ST) genome contigs; and LpTG-2 (NEA11) genome contigs. This result clearly shows a second variant of this gene in the NEA11 genome that has far more SNPs than the first NEA11 contig hit. This presumably represents the *E*. *typhina* copy of this gene that has been retained in the NEA11 genome. It is unlikely that this is a localised duplication in NEA11 as neither *E. festucae,* nor *N. lolii* has such a duplication.
**1: *E. festucae* (E2368) genome contigs**
**2: *N. lolii* (ST) genome contigs**
**3: LpTG-2 (NEA11) genome contigs**

The panel below shows the *N. lolii* peramine gene from GenBank used as a query against NEA11 genome assembly contigs. BLAST(N) alignment of *Lp*TG-2 endophyte strain NEA11 reads against the peramine gene (*perA*) sequence (GenBank accession number: AB205145). The presence of SNP in one set of contigs indicates the presence of two copies of the peramine gene sequence in endophyte strain NEA11.

### Mating-type analysis

In heterothallic fungi, such as *Epichloë* spp, strains must be of opposite mating-type for sexual reproduction to proceed. In *Epichloë* spp, sexual development is regulated by alternative *MAT1-1* (comprising *MAT1-1-1, MAT1-1-2* and *MAT1-1-3*) and MAT1-2 (comprising MAT1-2-1) genes at the *MAT* locus. Although the flanking regions of *MAT1-1* and *MAT1-2* are homologous, the nucleotide sequences of *MAT1-1* and *MAT1-2* idiomorphs are highly dissimilar (Figure 7).

The mating-type locus of *E. festucae* E2368 was contained in contig 5 of the original assembly (University of Kentucky, http://www.genome.ou.edu.fungi.html). This contig was aligned with contigs derived from *N. lolii* endophyte strain ST. The MAT1-1 mating-type locus genes found in *E. festucae* (*MAT1-1-1, MAT1-1-2, MAT1-1-3*) were demonstrated to be absent in the *N. lolii* consensus sequence (Figure 7). In the corresponding location a single gene for the opposite mating type (MAT1-2) was identified. This opposite mating type gene (*MAT1-2-1*) was found in all the *N. lolii* strains sequenced as well as NEA12 (Table 10).

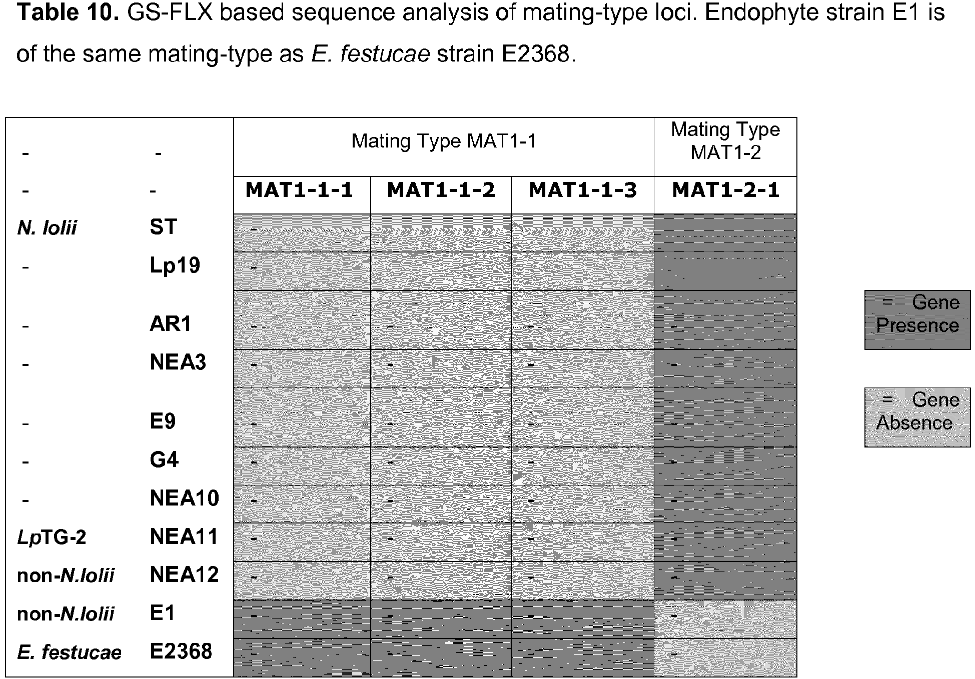

To assess the mating type of endophyte strain E1, the two possible mating type contigs were compared to E1 contigs. This activity proved that E1 contained the same three (*MAT1-1-1, MAT1-1-2, MAT1-1-3*) mating-type genes as *E. festucae* E2368 and is thus of the MAT1-1 mating-type. This is in contrast to the mating type gene of non-*N*. *lolii* strain NEA12, which is of the MAT1-2, *N. lolii*-like, mating-type.

Cluster analysis based on sequence nucleotide diversity shows that endophyte strains E1 and NEA12 cluster with *E. festucae* strain E2368, with their position in the tree switching between analysis based on the mating-type loci flanking sequence and the NoxR gene respectively, and suggesting that recombination has occurred in these lineages (Figure 8).

The identification of an endophyte strain of the opposite mating-type to previously characterised perennial ryegrass endophyte strains provides a means for molecular breeding of endophytes to deliver favourable traits into the plant endophyte symbiotum through the use of the novel E1 strain endophyte.

### Mitochondrial genome analysis

The mitochondrial genome of *N. lolii* endophyte strain Lp19 was present as a single c.88.7 kb contig. This sequence was used to identify contigs containing mitochondrial DNA sequences in the other *N. lolii* strains sequenced through BLAST(N)-based sequence similarity. Homology searches identified mitochondrial contigs in the *E*. *festucae* strain E2368 assembly the two non-*N*. *lolii* genomes and the L*p*TG-2 genome that were sequenced.

The mitochondrial genome sizes for each of the fungal endophytes sequenced in this study as well as the *E. festucae* strain E2368 are shown on Table 11. A representative of the Clavicipitaceae, *Metarhizium anisopliae* (Genbank reference number NC_008068.1), is shown for comparison. The *N. lolii* mitochondrial genomes are similar in size, ranging from 88,377 bp for G4 to 88,740 bp for AR1. L*p*TG-2 representative, NEA11 has a mitochondrion genome similar in size to *N. lolii.* The two non*-N.lolii* genomes, E1 (63,218 bp) and NEA12 (57,818 bp), have relatively smaller mitochondrial genomes more similar in size to that of *E. festucae* strain E2368 (69,614 bp) than that of *N. lolii.*

**Table 11. Mitochondrial genome size of the 10 fungal endophyte strains sequenced in this study, E. festucae strain E2368 and Metarhizium anisopliae.**

| | ***N. lolii* Lp19** | ***N. lolii* ST** | ***N. lolii* NEA3** | ***N. lolii* AR1** | ***N. lolii* E9** | ***N. lolii* G4** | ***N. lolii* NEA10** | **non-*N.lolii* E1** | **non-*N.lolii* NEA12** | **L*p*TG-2 NEA11** | ***Epichloë festucae 2368*** | ***Metarhizium anisopliae*** |
|---|---|---|---|---|---|---|---|---|---|---|---|---|
| Approximate Mitochondrial Genome Lengths (bp) | 88709 | 88711 | 87526 | 88740 | 88738 | 88377 | 88734 | 63219 | 57818 | 88692 | 69614 | 24674 |

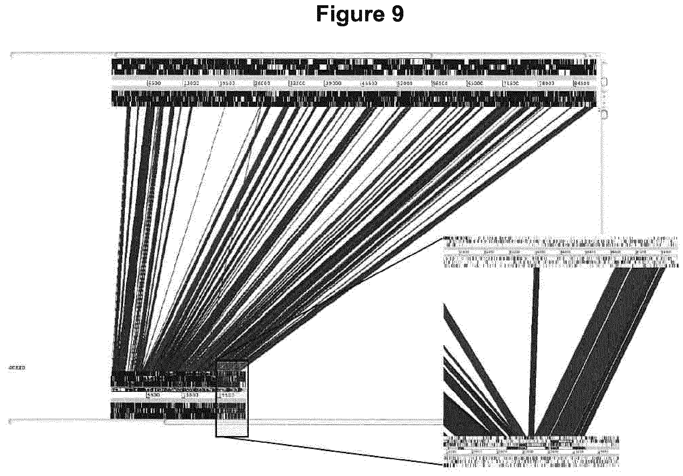

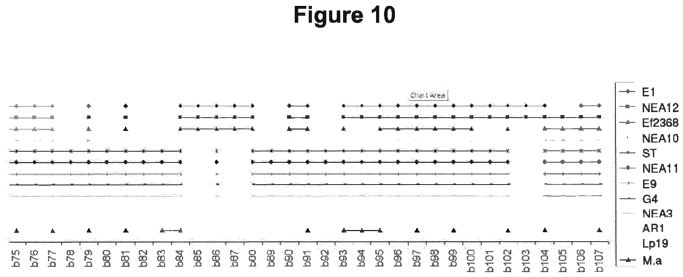

The multiple mitochondrial DNA sequences were used to generate a mitochondrial genome alignment along with the mitochondrial genome sequence of the Clavicipitaceae fungus *Metarhizium anisopliae.* The alignment demonstrated that while the different mitochondrial genomes vary in size, the genes are present in the same order and strand sense in all genomes, with differences being due to variable insertions in each strain (Figures 9 and 10).

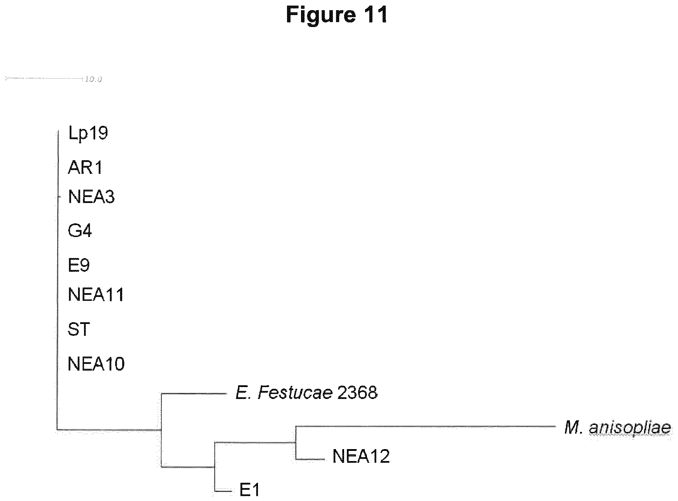

Scoring block presence as 1 and absence as 0, a matrix was created to generate a parsimony tree of the relationships between the mitochondrial genomes (Figure 11). This tree places the E1 and NEA12 mitochondria on a branch with the *E. festucae* strain E2368 mitochondrial genome, these three genomes showing greater variation than that of the *N. lolii* mitochondria. The mitochondrial tree shows that endophyte strains NEA12 and E1 are neither *E. festucae* nor *N. lolii,* but are more similar to *E. festucae* than *N*. *lolii.* Endophyte L*p*TG-2 NEA11 has a mitochondrial genome that is genetically a *N. lolii* type, being in a clade with NEA3 and AR1, within the *N. lolii* cluster.

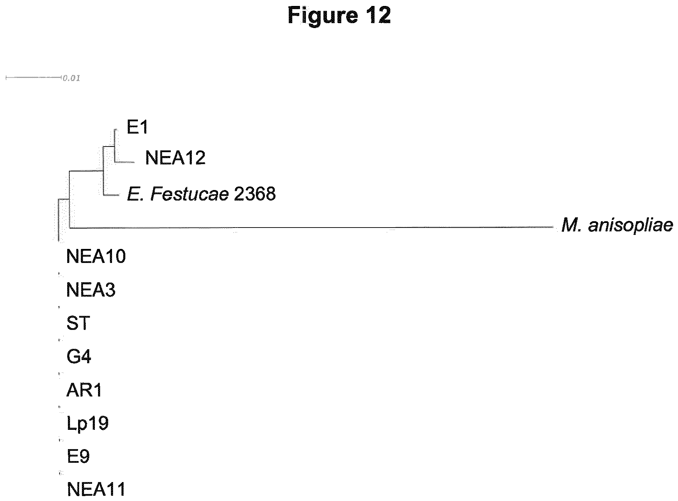

A similar pattern is observed if a neighbour joining tree is constructed using ClustalW from a DNA alignment of only the 40 blocks of sequence that are shared across all endophyte species (c. 40kb; Figure 12). There are still gaps present in the *Metarhizium anisopliae* sequence in this alignment.

### A quantitative PCR method for assaying endophyte biomass in planta

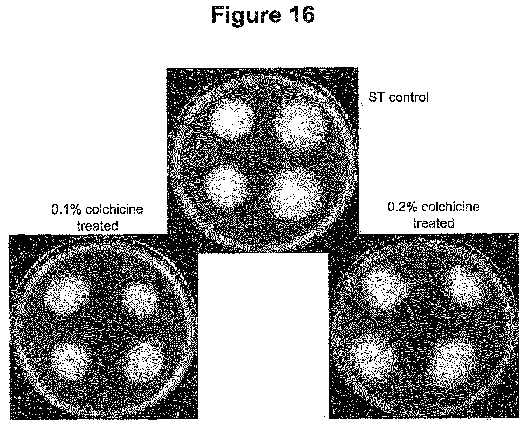

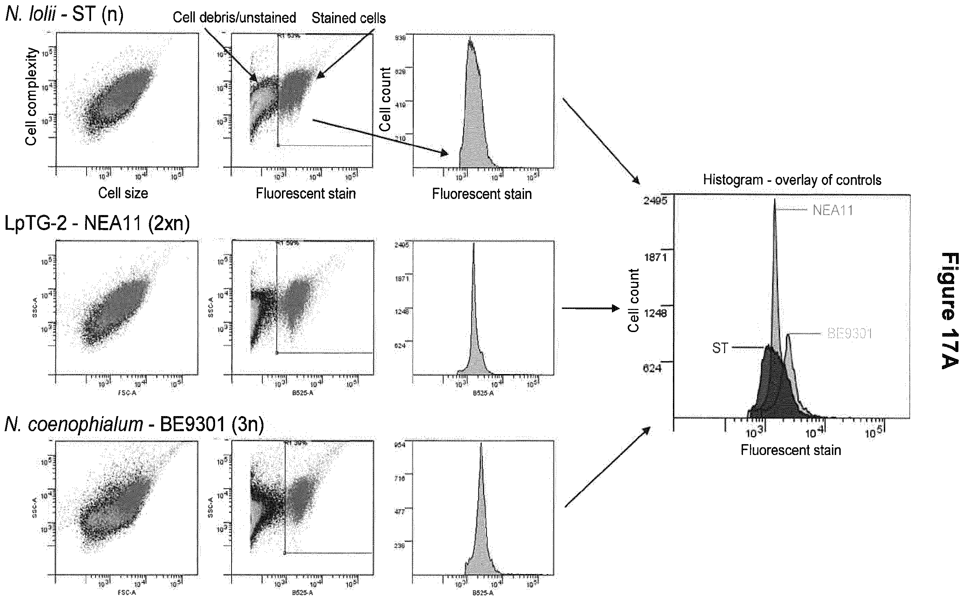

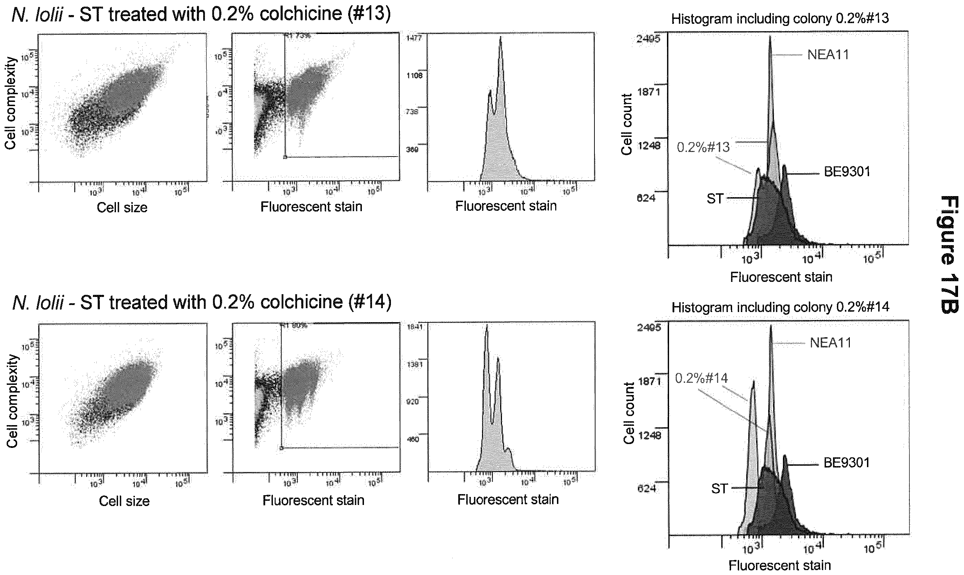

A quantitative PCR (qPCR) method for assaying endophyte biomass *in planta* has been developed and successfully implemented. The development of a high-throughput PCR-based assay to measure endophyte biomass *in planta* enables efficient screening of large numbers of plants to study endophyte-ryegrass biomass associations. qPCR-specific primer sets have been designed for the peramine biosynthesis gene (*perA*). To quantitatively assess *in planta* endophyte biomass, a standard curve, ranging from 2 x 10² to 2 x 10⁶ copies of the target sequence, has been generated from endophyte DNA template (Figure 16). The standard curve is used to quantitatively determine *in planta* endophyte biomass of unknown samples (Figure 17).

A proof-of-concept study was conducted using a subset of plants which had been previously analysed using established SSR methodology. The analysis clearly shows a correlation between the quantitative SSR allele scoring and the presence of endophyte *in planta* (Table 12).

**Table 12. Association between SSR-based analysis of endophyte presence and endophyte colonisation as determined by qPCR-based analysis. Each host genotype-endophyte combination represented three independent biological replicates. An SSR-based quality score was used to assess endophyte presence, a score of 3 indicated 3 out of 3 SSR markers were efficiently amplified and of the correct size.**

| ***Host plant-endophyte combination*** | ***SSR-based assay*** | ***qPCR results (copies*/*ng gDNA)*** |
|---|---|---|
| 1 | 1 | Negative |
| 2 | 3 | 16.638 |
| 3 | 3 | 68.98 |
| 4 | 1 | Negative/very low |
| 5 | 3 | 24.3 |
| 6 | 3 | 1.48 |
| 7 | 3 | 14.386 |
| 8 | 2 | 0.7646 |

### Example 4 - Molecular breeding - E1 as a vehicle for trait delivery into perennial ryegrass by hyper-inoculation

- Endophyte E1 is a genetically novel, non-*Neotyphodium lolii* endophyte. E1 is representative of an as yet un-named taxon
- This supposition is supported by mitochondrial and nuclear genome sequence analysis
- On the basis of DNA specific content, the predicted alkaloid profile of E1 indicates that the lolitrem B toxins deleterious to animal health are not produced by this endophyte.
- The E1 endophyte does not produce lolitrem B, ergovaline, peramine, lolines or janthitrems *in planta.*
- Endophyte E1 has the mating-type MAT1-1, the opposite mating-type to that carried by all *N. lolii* endophytes previously characterised
- Endophyte E1 has a high inoculation success rate in perennial ryegrass as compared to other endophytes
- The identification of an endophyte of the opposite mating-type that is highly compatible and stable *in planta* provides a means for molecular breeding of endophytes for perennial ryegrass through hyper- inoculation

Hyphal fusion between endophyte strains of the opposite mating-type provides a means for delivery of favourable traits into the host plant via hyper-inoculation. Such strains would include: 1) an endophyte strain that exhibits the favourable characteristics of high inoculation frequency and high compatibility with a wide range of elite perennial ryegrass host germplasm and; 2) an endophyte that exhibits a low inoculation frequency and low compatibility, but has a highly favourable alkaloid toxin profile.

The E1 endophyte strain is genetically novel and is compatible with a wide range of elite germplasm as it can be inoculated with a high degree of success. E1 also is of the opposite mating-type to all of the previously characterised perennial ryegrass endophytes. Molecular breeding may therefore be applied by combining the highly compatible E1 endophyte traits with the favourable toxin profile traits of endophytes such as NEA12.

The process of molecular breeding through vegetative (hyphal) fusion may occur *in planta* by co-inoculation of two endophyte into the same plant. However, molecular breeding may be more efficiently achieved through vegetative fusion in *in vitro* culture of endophytes of the opposite mating-type, followed by hyper-inoculation of the resultant endophyte.

The following experimental design is applied for molecular breeding of fungal endophytes
1. Determine vegetative compatibility of known endophytes using established co-culturing methodologies.
2. Generation of auxotrophic mutants (e.g. by gene silencing techniques such as RNAi) for two strains of endophyte, such as E1 and NEA12, exhibiting opposite mating-types.
3. Development of vegetative (hyphal) fusion protocol using a combination of cell well degrading enzymes and PEG-4000.
4. Screen for regenerated endophytes based on survival (indicating complementarity of auxotrophic mutations).
5. Genetic screen using SSR and/or mating-type markers to confirm presence of the hybrid genome in a single nuclear compartment.
6. Inoculation and compatibility/stability assessment of endophytes using established methodologies.
7. Phenotypic assessment of endophyte-host associations using established methodologies.

### Example 5 - Generation of artificial polyploids of fungal endophytes

Colchicine has been widely used for induction of polyploidy in plant species such as perennial ryegrass, as compared to the application to fungi, which has been limited to a few species.

The mitotic spindle inhibitor colchicine is capable of inducing autopolyploidisation, and may be applicable to the production of artificial polyploid endophytes.

Artificial polyploids were generated by colchicine induced chromosome doubling of the endophyte strains ST and NEA12.

NEA12, a janthitrem only producing endophyte, with superior bioprotective properties forms stable associations with a limited range of perennial ryegrass hosts. An artificial polyploid of NEA12 that is non-toxic to mammals, with enhanced bioprotective properties, that is broadly compatible and highly stable is highly desirable to industry.

### Generation of artificial polyploids

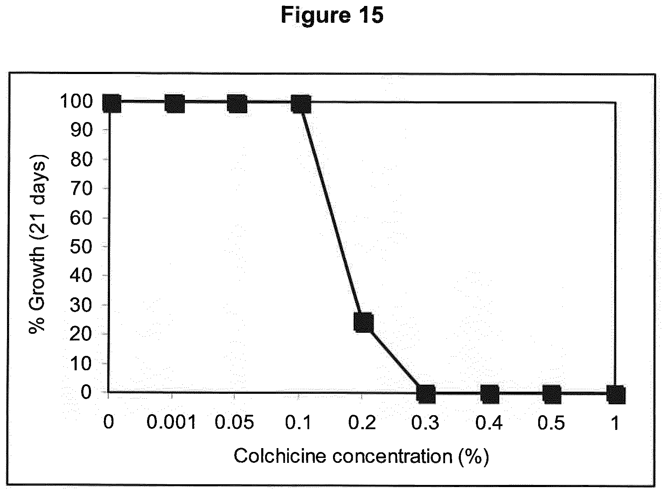

Experiments were conducted to determine the range of colchicine concentrations in which the mycelia of the fungal endophyte *N. lolii* (strain ST) would grow successfully. Mycelia were grown in colchicine concentrations ranging from 0% to 1% for 21 days and monitored for growth (Figure 15). At greater than or equal to 0.2% colchicine mycelium growth halted whereas at 0.1% or less colchicine mycelium growth was prolific.

Artificial polyploids were generated for endophyte strains ST and NEA12. Endophyte strains ST and NEA12 (n) were grown in 0, 0.1 and 0.2% colchicine and potato dextrose broth for 21 days followed by a 7-10 day recovery period in potato dextrose broth only. Protoplasts were generated from all colchicine concentrations and single colonies isolated (Figure 16).

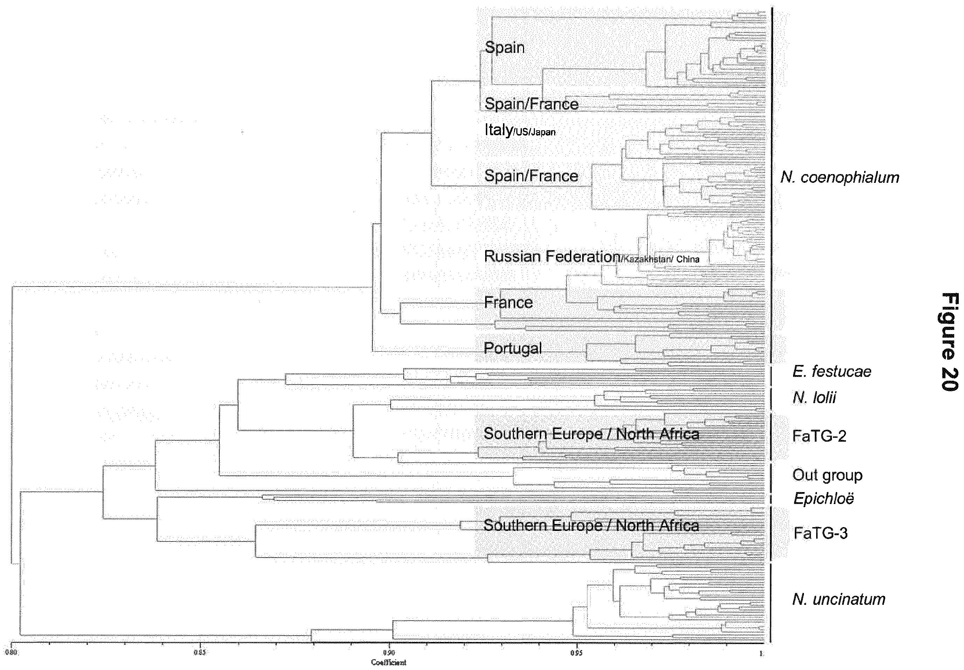

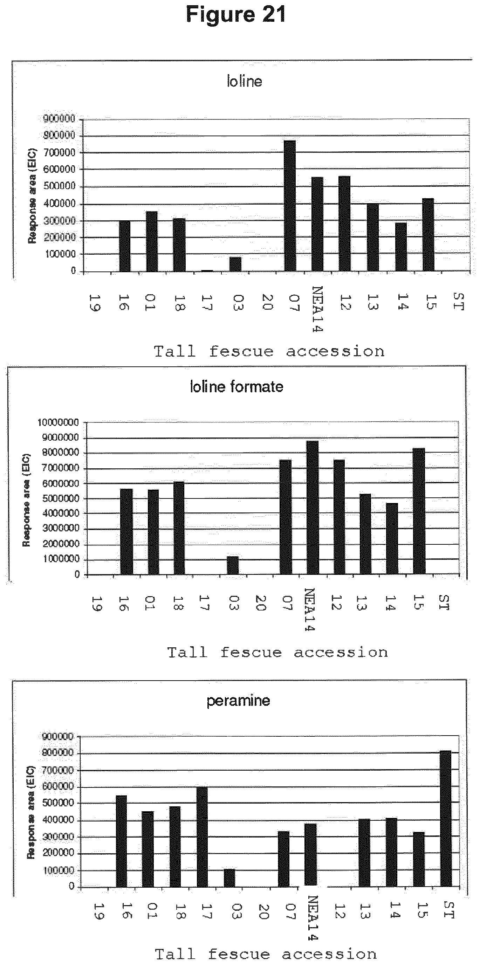

*N. coenophialum* strain BE9301 and LpTG-2 strain NEA11 which are natural heteroploids (3n and 2xn fused respectively) have been utilised as control material for assessment of ploidy changes using flow cytometry. An optimised protocol was established allowing analysis of fungal protoplasts via flow cytometry. A number of colonies have been identified with changes in nuclear DNA content relative to the control samples (Figures 20 and 21).

**Table 13: Summary of individual endophyte colonies, ST and NEA12, treated with colchicine and subjected to flow cytometry analysis.**

| **Endophyte** | **Colchicine treatment (%)** | **Number of colonies** | **Number colonies analysed** |
|---|---|---|---|
| *N.lolii* ST | 0.2 | 12 | 12 |
| *N.lolii* NEA12 | 0.1 | 60 | 2 |
| *N.lolii* NEA12 | 0.2 | 60 | 18 |

### Example 6 - Generation of novel endophyte variation using ionising radiation

### Summary

- Lolitrem B is the major alkaloid leading to ryegrass staggers in grazing animals.
- A method has been developed to eliminate the production of the detrimental alkaloid lolitrem B, using X-ray mutagenesis induced deletion of genes in the lolitrem B biosynthetic gene cluster, in the ST endophyte.
- Such an endophyte would be advantageous over existing commercial endophytes, as ST is highly stable and broadly compatible.

### Introduction

Ionising radiation is capable of introducing a broad range of mutagenic lesions and has been found to be very effective in many species. Published methods are available to readily detect deletion mutants in targeted plant genes (Li *et al*, 2002). Experiments have been performed to determine if *N. lolii* mycelia are amenable to production of mutagenic lesions by ionising radiation, in particular deletion mutations.

### Generation of novel endophyte variation using ionising radiation

*N. lolii* strain ST was grown in potato dextrose broth for different periods of time ranging from 2-14 days before exposure to ionising radiation. Radiation from a caesium source was applied to the liquid cultures in doses ranging from 10-30 Gy. Following a recovery period (10-14 days) the radiation dose was repeated. Protoplasts were generated and recovery of individual colonies monitored over a 4-6 week period.

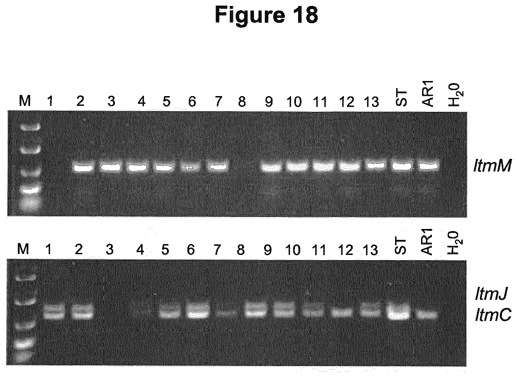

Lolitrem B is the major alkaloid leading to ryegrass staggers in grazing animals. Three genes within the lolitrem B gene cluster, which contains 10 genes all required for synthesis of lolitrem B, were targeted to identify individual *N. lolii* colonies with deletions (Young *et al*, 2005). A high throughput PCR screening method was developed to detect for the presence and absence of the three lolitrem B genes (Figure 18).

**Table 14: Analysis of ionising radiation experiments. Protoplast regeneration, concentration of recovered protoplasts and number of PCR analysed colonies.**

| **Endophyte strain** | **Age of Culture** | **Dose (Gy)** | **Irradiation events** | **Protoplast regeneration** | **Concentration** | **Colonies plated** | **PCR screened colonies** |
|---|---|---|---|---|---|---|---|
| ST | 2 wks | 0 | 1 | ✔ | 1.8x10⁸ pp/ml | - | - |
| ST | 2 wks | 10 | 1 | ✔ | 5.8x10⁵ pp/ml | 700 | 450 |
| ST | 2 wks | 15 | 1 | ✔ | 7.5x10⁵ pp/ml | 200 | 200 |
| ST | 2 wks | 20 | 1 | ✔ | 2.2x10⁶ pp/ml | 2950 | 400 |
| ST | 2 wks | 30* | 1 | - | - | - | - |
| ST | 2 wks | 30 | 1 | ✔ | 1.94x10⁷ pp/ml | 400 | 350 |
| | | | | | | | |
| ST | 2 wks | 0 | 2 | ✔ | 1.1x10⁸ pp/ml | - | - |
| ST | 2 wks | 10 | 2 | ✔ | 2.6x10⁵ pp/ml | 150 | 150 |
| ST | 2 wks | 15 | 2 | slow/reduced numbers | 2.2x10⁷ pp/ml | 200 | 200 |
| ST | 2 wks | 20 | 2 | ✔ | 1.38x10⁷ pp/ml | - | - |
| ST | 2 wks | 30/25 | 2 | ✔ | 6.38x10⁵ pp/ml | 1000 | 750 |
| ST | 2 wks | 30 | 2 | ✔ | 1.3 x 10⁸ pp/ml | 900 | 300 |
| | | | | | | | |
| ST | 4 Days | 0 | 1 | ✔ | 2.5x10⁸ pp/ml | - | |
| ST | 4 Days | 10 | 1 | slow/reduced numbers | 3.75x10⁸ pp/ml | 200 | 200 |
| ST | 4 Days | 15 | 1 | slow/reduced numbers | 1.38x10⁸ pp/ml | 200 | 200 |
| ST | 4 Days | 20 | 1 | slow/reduced numbers | 2.7x10⁵ pp/ml | - | - |
| ST | 4 Days | 25 | 1 | slow/reduced numbers | 1.38x10⁵ pp/ml | 50 | 50 |
| ST | 4 Days | 30 | 1 | slow/reduced numbers | 1.38x10⁷ pp/ml | - | - |
| *30 Gy dose for first irradiation | | | | | Total | 6950 | 3250 |

### Example 7 - Tall fescue endophyte discovery and characterisation

### Summary

### Tall fescue endophyte discovery

The strategies implemented for perennial ryegrass endophyte discovery were extended to the resident endophytes of tall fescue (including the *Fa*TG-2 and *Fa*TG-3 taxonomic groups).

A targeted collection of tall fescue germplasm was made from throughout the range of natural growth and domesticated cultivation.

A total of 568 tall fescue accessions obtained from 40 different countries were tested for endophyte incidence using endophyte-specific simple sequence repeat (SSR) genetic markers. Twelve to twenty seeds from each accession were tested for endophyte presence. Total genomic DNA was extracted from two independent seed bulks of 6-10 seeds from each accession and endophytes were detected by PCR amplification with six endophyte-specific SSR markers.

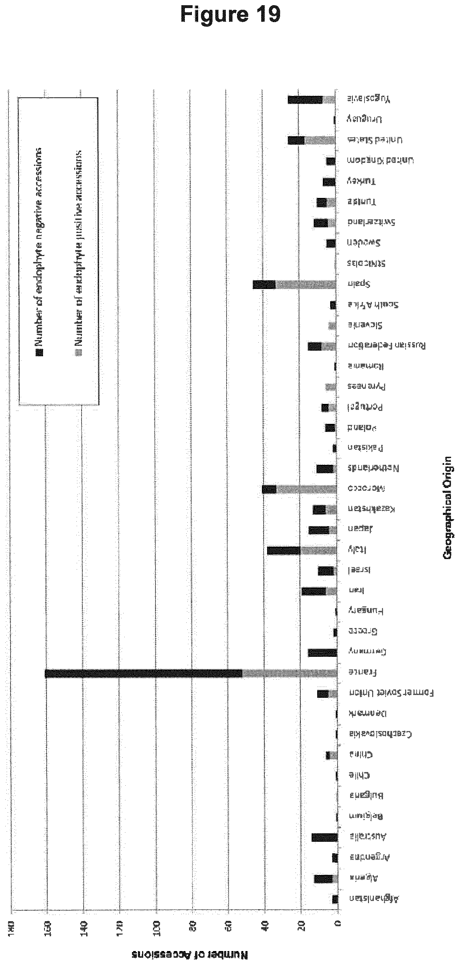

Endophyte was detected in 40% (228/568) of the tall fescue accessions tested. Furthermore, accessions from 23 out of the 40 countries screened were endophyte positive (Figure 19) showing the highest incidence in Morocco and Pyrenees, where the majority of accessions tested (80%-100%) were endophyte positive. Accessions originating from Italy, Spain, and United States exhibited a higher endophyte incidence among the tall fescue accessions tested.

A subset of selected endophyte positive samples, were selected for further analysis using 32 endophyte-specific SSR markers. The selected genotypes represent a broad range of known geographical origins, hence representing an effective survey of tall fescue endophyte genotypic variation. A set of 52 reference isolates representing several endophyte species, including the resident endophyte of tall fescue and meadow fescue were also included to the diversity analysis.

The UPGMA phenogram, constructed using average taxonomic distance based on SSR polymorphism across 203 endophyte positive accessions, represented six different known taxa, and two out-grouped clusters (Figure 20). The phenogram was supported by Mantel test statistics showing a high correlation coefficient (r = 0.95) which indicated a high goodness-of-fit for the data. Endophytes representing six different taxa were detected in the 203 accessions (Figure 20). The majority of endophytes (60%; 122/203) appeared to belong to the taxon *Neotyphodium coenophialum,* clustering in the phenogram with *N. coenophialum* isolates from the reference endophyte collection (Figure 20). This species occurred in 72% (122/170) of tall fescue collection accessions.

As defined by the *N. coenophialum* reference isolates, the *N. coenophialum* cluster comprised five main sub-clusters, of which the fifth sub-cluster is rather out grouped from the other four (Figure 20).

The genetic variation observed within *N. coenophialum* was high when comparing it with other taxonomic groups. In the phenogram *N. coenophialum* strains clustered for the most part according to their geographical origin (Figure 20). The first sub-cluster of *N*. *coenophialum* comprised mainly tall fescue accessions from Spain (28) and few accessions from Pyrenees (3) and France (4) (Figure 20). Italian (7) and French (14) accessions were clustered in the second sub-cluster (Figure 20). The third sub-cluster clearly shows the genetic similarity among accessions collected from geographic area surrounding Russian Federation [Slovenia (3), Russian Federation (6), Kazakhstan (7), Former Soviet Union (4) and China (3)] (Figure 20). Furthermore within the third sub-cluster a set of accessions from France (11) and Pyrenees (1) have formed a separate cluster from Russian Federation and its surrounding geographic origins. The fourth sub-cluster comprises only five endophytes of which two are Moroccan accessions and two are AR endophytes (AR542 and AR584) which were initially isolated from tall fescue originated in Morocco (Latch *et al*, 2000). The accessions collected from Portugal (4) have formed a distinct sub-cluster which is separated from all the other four sub-clusters (Figure 20).

FaTG-2 accessions formed a cluster close, but distinct from isolates of *N. lolii* (Figure 20). There were 20 FaTG-2 endophyte genotypes tall fescue collection which clustered with the *Fa*TG-2 reference genotype. Among them, a set of six accessions formed sub-clusters having lesser genetic similarity to the *Fa*TG-2 reference genotype. Therefore, the endophytes of those sub-clusters were named "*Fa*TG-2 like" endophyte genotypes.

A set of six endophyte genotypes formed a distinct cluster with putative *Fa*TG-3 reference isolates as defined by the previously-analysed AR endophytes. Furthermore, 13 accessions primarily originating from Morocco (9/13) formed a sub-cluster with putative *Fa*TG-3 isolates and those unidentified accessions, forming a cluster distinct to putative *Fa*TG-3 were named "*Fa*TG-3 like" endophytes (Figure 20).

The identities of selected putative *Fa*TG-2 and *Fa*TG-3 accessions are largely consistent with geographical provenance, as these taxa are known to be characteristic of populations from southern Europe and North Africa.

Two out grouped clusters were also identified and they were named as "out-group I" and "out-group II" (Figure 20). Accessions of Mediterranean origin primarily clustered in "out-group I", whereas one accession from Former Soviet Union formed the second out-group. Moreover, within "out-group I" Italian accessions clearly group separately from Moroccan and Algerian accessions.

A number of candidate novel endophytes have been identified.

### Metabolic profiling of tall fescue - endophyte associations

Representative tall fescue - endophyte associations were selected for metabolic profiling analysis in order to determine the endophyte derived alkaloid profile, in particular, lolitrem B, ergot alkaloids, peramine and lolines.

Analysis of metabolite production was assessed under controlled conditions using a growth chamber. Tall fescue - endophyte associations were each replicated four times by clonal splitting and arranged in a randomised block design in the growth chamber. Plants were maintained in soil for six weeks, with trimming every two weeks to encourage growth. Following 6 weeks growth, pseudostem tissue was harvested and freeze dried prior to performing a metabolite extraction and LCMS analysis. The perennial ryegrass- *N. lolii* designer association Bronsyn-ST was used as a control as ST is known to produce lolitrem B, ergovaline and peramine. For each of the accessions, the presence and identity of the resident endophyte was confirmed through SSR analysis of the plant material harvested for metabolic profile analysis and endophyte negative samples were removed from further analysis.

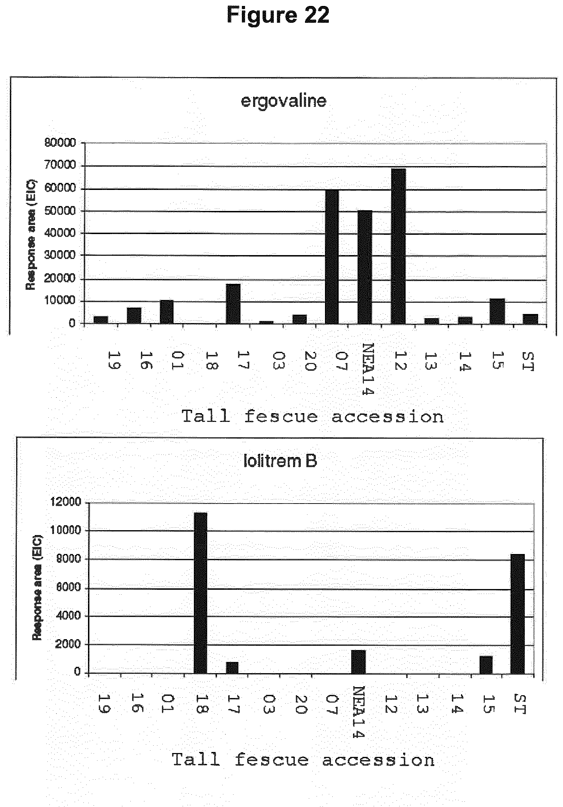

The results of the qualitative assessment alkaloid of production for 20 novel tall fescue endophytes are summarised in Table 15. Relative quantitation data for Batch three, comprising 13 endophytes assessed in their endogenous hosts, are shown in Figure 21 and Figure 22. A number of novel endophytes with favourable toxin profiles (low/no ergovaline production combined with loline and peramine production) have been identified.

**Table 15. Summary of alkaloid profiles for selected tall fescue endophytes in their endogenous host. *Relative quantitation of ergovaline levels: L=Low; M=Medium; H=High.**

| ***Tall fescue accession details*** | | ***Batch # for alkaloid profiling*** | ***Alkaloid profile*** | | | | ***Confirmed profile*** |
|---|---|---|---|---|---|---|---|
| ***Tall fescue accession*** | ***Endophyte species*** | | ***Lolines*** | ***Peramine*** | ***Ergovaline**** | ***Lolitrem B*** | |
| ***1*** | *N. coenophialum* | 1 & 3 | + | + | +^{M} | - | Y |
| ***NEA13*** | *N. coenophialum* | 2 | n.d | + | + | n.d | n.a |
| ***3*** | *N. coenophialum* | 3 | + | + | +^{L} | - | n.a |
| ***4*** | *N. coenophialum* | 1 | n.d | + | + | n.d | n.a |
| ***5*** | *N. coenophialum* | 2 | n.d | + | + | n.d | n.a |
| ***6*** | *N. coenophialum* | 2 | n.d | - | + | n.d | n.a |
| ***7*** | *N. coenophialum* | 2 & 3 | + | + | +^{H} | - | Y |
| ***8*** | *N. coenophialum* | 2 | n.d | + | + | n.d | n.a |
| ***9*** | *N. coenophialum* | 2 | n.d | + | + | n.d | n.a |
| ***10*** | *N. coenophialum* | 2 | n.d | - | + | n.d | n.a |
| ***NEA14*** | *N. coenophialum* | 1 & 3 | + | + | +^{H} | - | Y |
| ***12*** | *N. coenophialum* | 2 & 3 | + | - | +^{H} | - | Y |
| ***13*** | *N. coenophialum* | 1 & 3 | + | + | +^{L} | - | Y |
| ***14*** | *N. coenophialum* | 2 & 3 | + | + | +^{L} | - | Y |
| ***15*** | *N. coenophialum* | 1 & 3 | + | + | +^{M} | - | Y |
| ***16*** | *Fa*TG-2 | 3 | + | + | +^{M} | - | n.a |
| ***17*** | *Fa*TG-2 | 2 & 3 | - | + | +^{M} | - | N |
| ***18*** | *Fa*TG-3 | 3 | + | + | - | + | n.a |
| ***19*** | Out group 1 | 2 & 3 | - | - | +^{L} | - | Y |
| ***20*** | Out group 1 | 1 & 3 | - | - | +^{L} | - | Y |
| ***ST*** | *N. lolii* | 3 | - | + | + | + | Y |

### Establishment of meristem cultures for a diverse fescue host panel

Tissue culture responsive genotypes from selected germplasm material have been generated (Drover, Dovey, Bariane, Barolex). Table 16 shows the host cultivars, and their tissue culture responsive genotype, selected for further study. Each of the selected genotypes has a regeneration frequency greater than 80%

**Table 16. Establishment of meristem cultures for a diverse tall fescue host panel.**

| ***Cultivar*** | ***TCR genotype used for inoculation*** | ***Species*** | ***Characteristics*** |
|---|---|---|---|
| Bariane | BARI 27 | *L. arundinaceum* | Soft leaved, later maturing, highly palatable |
| Dovey | DOV 24 | *L. arundinaceum* | High yielding, fast establishing |
| Quantum | QUAN 17 | *L. arundinaceum* | Soft leaved with improved rust resistance |
| Jesup | JESS 01 | *L. arundinaceum* | Cool season perennial forage |
| Bronsyn | BRO 08 | *L. perenne* | Standard perennial ryegrass forage type |

### Tall fescue endophyte isolation

Selected novel endophytes were isolated from tall fescue accessions (Table 17).

**Table 17. Summary of endophytes isolated from tall fescue accessions**

| ***Endophyte Accession*** | ***Origin*** | ***Taxon*** |
|---|---|---|
| 1 | Spain | *N. coenophialum* |
| NEA13 | | *N. coenophialum* |
| 4 | Pyrenees | *N. coenophialum* |
| 5 | Pyrenees | *N. coenophialum* |
| 6 | Catalunya (Spain) | *N. coenophialum* |
| 7 | Corsica (France) | *N. coenophialum* |
| 8 | Corsica (France) | *N. coenophialum* |
| 9 | Corsica (France) | *N. coenophialum* |
| 10 | Aragon (Spain) | *N. coenophialum* |
| NEA14 | PaySardegna (France) | *N. coenophialum* |
| 12 | Aragon (Spain) | *N. coenophialum* |
| 13 | Gaurda (Portugal) | *N. coenophialum* |
| 14 | Gaurda (Portugal) | *N. coenophialum* |
| 15 | Aragon (Spain) | *N. coenophialum* |
| 17 | Spain | FaTG-2 |
| 18 | Tunisia | FaTG-3 |
| 19 | Algeria | outgroup1 |
| 20 | Sardegna (NW Italy) | outgroup1 |
| 21 | Catalunya (Spain) | *N. coenophialum* |

### Isogenic inoculation of novel tall fescue endophytes

A set of ten novel tall fescue endophytes were selected for inoculation based on genetic novelty using SSR-based diversity analysis and the toxin profile based on qualitative metabolic profiling (Table 18). Included in the set was the endophyte AR542 a commercial endophyte in use globally. AR542 was discovered and isolated by AgResearch NZ and is marketed as MaxP™ and MaxQ™.

**Table 18. Endophytes selected for isogenic inoculation based on analysis of genetic diversity and metabolic profile**

| ***Tall fescue accession details*** | | ***Alkaloid profile*** | | | |
|---|---|---|---|---|---|
| ***Tall fescue accession*** | ***Endophyte species*** | ***Lolines*** | ***Peramine*** | ***Ergovaline*** | ***Lolitrem B*** |
| ***NEA13*** | *N. coenophialum* | n.d | + | + | n.d |
| ***3*** | *N. coenophialum* | + | + | +^{L} | - |
| ***22*** | *N. coenophialum* | n.d | n.d | n.d | n.d |
| ***NEA14*** | *N. coenophialum* | + | + | +^{H} | - |
| ***13*** | *N. coenophialum* | + | + | +^{L} | - |
| ***15*** | *N. coenophialum* | + | + | +^{M} | - |
| ***17*** | F*a*TG-2 | - | + | +^{M} | - |
| ***19*** | Out group 1 | - | - | +^{L} | - |
| ***20*** | Out group 1 | - | - | +^{L} | - |
| ***AR542**** | *N. coenophialum* | n.d | n.d | - | n.d |

| | | | | | |
|---|---|---|---|---|---|
| *toxin profile from Bouton *et al,* 2002. | | | | | |

In order to accurately determine the phenotypic effects of different candidate endophytes in the absence of host-specific genetic effects, a system for isogenic inoculation was used. Novel candidate endophytes were individually inoculated into elite tall fescue germplasm as well as the perennial ryegrass host genotype Bronsyn (Bro08). Following inoculation and plantlet regeneration in culture, plants were transferred to soil for three months to allow establishment of endophyte and host-plant associations. After this period, three tillers from each plant were sampled and tested for endophyte presence using SSR-based analysis.

Of the 498 isogenic inoculations tested, 109 (21.9%) could be positively scored with a high degree of confidence. Successful inoculations are listed on Table 19.

Variation in inoculation success according to candidate endophyte identity was observed. Endophyte strain 3 (4.3%), for example, exhibited relatively lower success rates as compared to strain 20 (51.1%), or the commercial endophyte AR542 (44.4%; Table 19) and only formed stable associations with one of the five hosts (Bariane). No successful inoculations were identified for endophyte strain 15. FaTG-2 endophyte, strain 17, is a highly compatible endophyte which obtains a high rate of success of inoculation into tall fescue (Table 19) compared to other endophytes examined, and is comparable to AR542. Out-group 1 endophyte strain 20 exhibits the highest level of compatibility as measured by its ability to be inoculated.

Both tall fescue endophytes inoculated into perennial ryegrass host Bro08, strain NEA13 and strain NEA14, were taken up successfully, establishing that endophyte inoculation across a range of host species is possible.

**Table 19. Summary statistics for isogenic inoculations of selected candidate endophytes in a targeted isogenic tall fescue and perennial ryegrass panel of 5 hosts.**

| **C. Percent of successful inoculations** | | | | | | | | | | | |
|---|---|---|---|---|---|---|---|---|---|---|---|
| | ***Endophyte strain*** | | | | | | | | | | |
| ***Host plant genotype*** | ***22*** | ***3*** | ***NEA13*** | ***15*** | ***NEA14*** | ***AR542*** | ***13*** | ***17*** | ***20*** | ***19*** | ***Total*** |
| ***BARI24*** | 13.0 | 12.5 | 22.2 | 0.0 | 0.0 | 42.3 | 16.7 | 56.5 | 54.5 | 8.3 | 24.3 |
| ***BRO 08*** | TBD | TBD | 18.2 | TBD | 11.8 | TBD | TBD | TBD | TBD | TBD | 14.3 |
| ***DOV 24*** | 30.0 | 0.0 | TBD | TBD | TBD | TBD | TBD | TBD | TBD | TBD | 12.5 |
| ***JESS 01*** | 30.4 | 0.0 | 17.9 | 0.0 | 35.0 | 47.4 | 20.0 | 10.0 | 41.7 | 20.0 | 22.2 |
| ***QUAN 17*** | 37.5 | 0.0 | 10.0 | 0.0 | TBD | TBD | TBD | TBD | TBD | TBD | 17.5 |
| ***Total*** | 25.0 | 4.3 | 17.9 | 0.0 | 13.4 | 44.4 | 18.2 | 41.5 | 51.1 | 13.6 | **21.9** |
| **Species** | | | ***N. coenophialum*** | | | | | ***Fa* TG-2** | **Outgroup 1** | | |
| TBD | Be Determined | | | | | | | | | | |

### Example 8 - Antifungal activity of Neotyphodium/Epichioë endophytes

### Introduction

*Neotyphodium* endophytes at present are largely unexplored in terms of their production of novel antimicrobials.

While some *Epichloë*/*Neotyphodium* endophytes have been shown to inhibit the growth of plant-pathogenic fungi *in vitro,* the inhibitory substances produced have not been identified.

Endophytes with anti-fungal properties may benefit host plants by preventing pathogenic organisms from colonising them and causing disease. This is of particular interest to the turf grass industry.

### A bioassay to assess antifungal activity of Neotyphodium endophytes

To determine if endophytes of the species *Neotyphodium* produce anti-fungal substances *in vitro* representative species/strains from *Neotyphodium* were tested for the presence of anti-fungal activity against eight species of fungal plant pathogens.

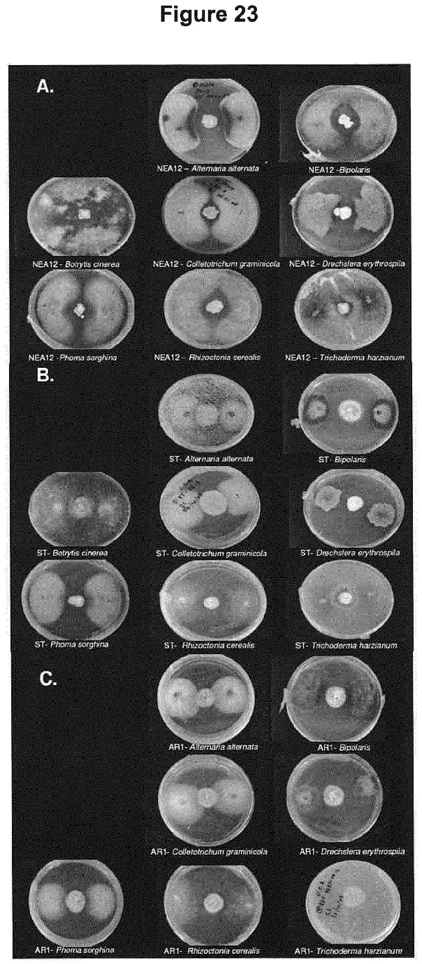

Three types of inhibition reactions were observed. In the first reaction, pathogenic fungal growth was unaffected. In the second, growth of the pathogenic fungi was initially unaffected, but growth ceased when the colony margin approached a "critical" distance from the central endophyte colony. In the third stronger reaction type, the overall growth of the colony of the pathogenic fungi was reduced. Examples of inhibition reactions are shown in Figure 23.

Variation was observed within and between endophyte taxa. Non-*N*. *lolii* strain NEA12 exhibits the strongest and most broad spectrum antifungal activity. Variation was also observed among genetically distinct strains of *N. lolii.* Within *N. lolii,* strains with strongest to weakest effects were ST > AR1 > NEA3 > NEA10. ST exhibited the broadest spectrum of antifungal activity, inhibiting the growth of 7/8 fungi strains tested.

The bioassay results showed that endophytes *in vitro* exhibit variation in anti-fungal activity that does not correlate with known toxin production (specifically, lolitrem B, ergovaline and peramine). For example NEA12 does not produce lolitrem B, ergovaline and peramine and has strong antifungal activity and ST does produce lolitrem B, ergovaline and peramine and also has strong antifungal activity.

**Table 20. Antifungal activity exhibited by representative strains of N. lolii and related endophyte taxa. Assays were scored visually from 0 - 5. NT- not tested.**

| | | ***Fungal species*** | | | | | | | |
|---|---|---|---|---|---|---|---|---|---|
| ***Endophyte strain*** | ***Endophyte species*** | ***Alternaria alternata*** | ***Colletrichum graminicola*** | ***Rhizoctonia cerealis*** | ***Trichoderma harzianum*** | ***Phomasorghina*** | ***Botrytis cinerea*** | ***Bipolaris portulaceae*** | ***Drechslera brizae*** |
| **AR510** | ***Fa*TG-3** | 0 | 0 | 5 | 1 | NT | NT | NT | NT |
| **NEA11** | **L*p*TG-2** | 0 | 1 | 2 | 0 | NT | NT | NT | NT |
| **AR1** | ***N. lolii*** | 0 | 0 | 3 | 0 | 2 | 0 | 1 | 1 |
| **NEA10** | ***N. lolii*** | 0 | 0 | 0 | 0 | 0 | 0 | 1 | 1 |
| **NEA3** | ***N. lolii*** | 0 | 0 | 1 | 1 | 1 | 0 | 0 | 0 |
| **ST** | ***N. lolii*** | 0 | 1 | 3 | 2 | 2 | 2 | 4 | 3 |
| **NEA12** | **Non-*N*. *lolii*** | 3 | 4 | 4 | 3 | 3 | 3 | 3 | 2 |

| | | | | | | | | | |
|---|---|---|---|---|---|---|---|---|---|
| Samples are scored visually from 0-5. 0 is no antifungal activity, 1 is low antifungal activity, 5 is strong antifungal activity. NT- not tested | | | | | | | | | |

### Mass spectrometry for identification of antifungal metabolites

Mass spectrometry was used to determine the relationship between antifungal activity and metabolite expression.

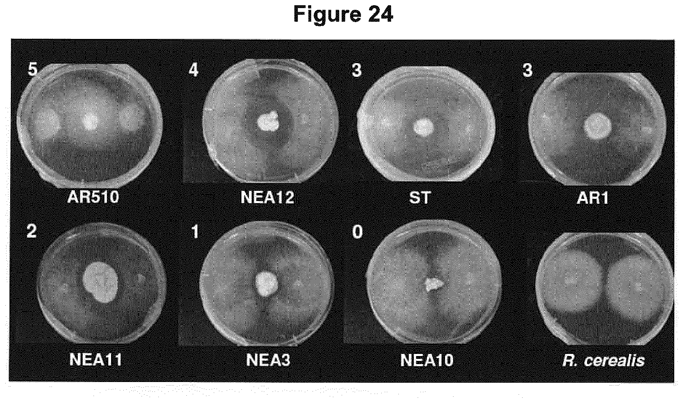

Endophyte strains representing the full spectrum of antifungal activity were selected for analysis in order to identify those alkaloids that may be associated with antifungal activity (Figure 24).

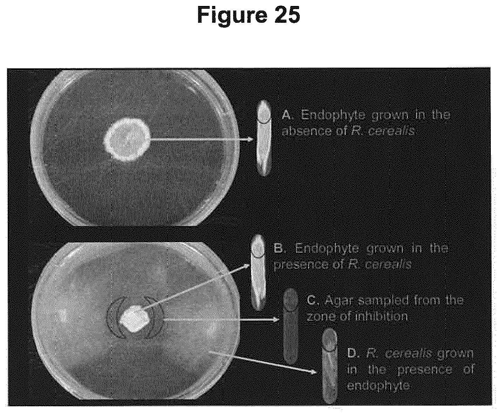

Endophyte strains were grown both in the presence and absence of the pathogenic fungi *Rhizoctonia cerealis* (Figure 25). Freeze dried endophyte mycelia was then extracted for metabolic profiling analysis.

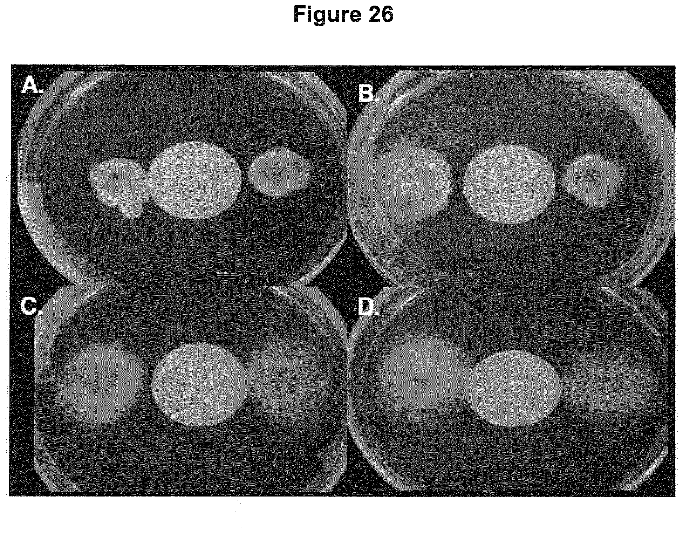

Following extraction, a validation assay was done to ensure that the alkaloids associated with antifungal activity had been appropriately extracted (Figure 26). The antifungal activity of the extract used for LCMS analysis was confirmed. The expression of antifungal alkaloids is constitutive as extracts taken from endophyte in the absence of *Rhizoctonia cerealis* also exhibit antifungal activity (Figure 26).

### Example 9 - Metabolic profiling

### Summary

Perennial ryegrass cultivars inoculated with the NEA12 endophyte were analysed using LCMS. The toxins peramine, ergovaline and lolitrem B were not detected in the extract. The AR37 metabolite 11,12-epoxy janthitrem G was detected and its structure assigned based on retention time and MS analysis of an extract of the AR37 inoculated perennial ryegrass.

### Metabolic profiling of endophyte NEA12 in perennial ryegrass.

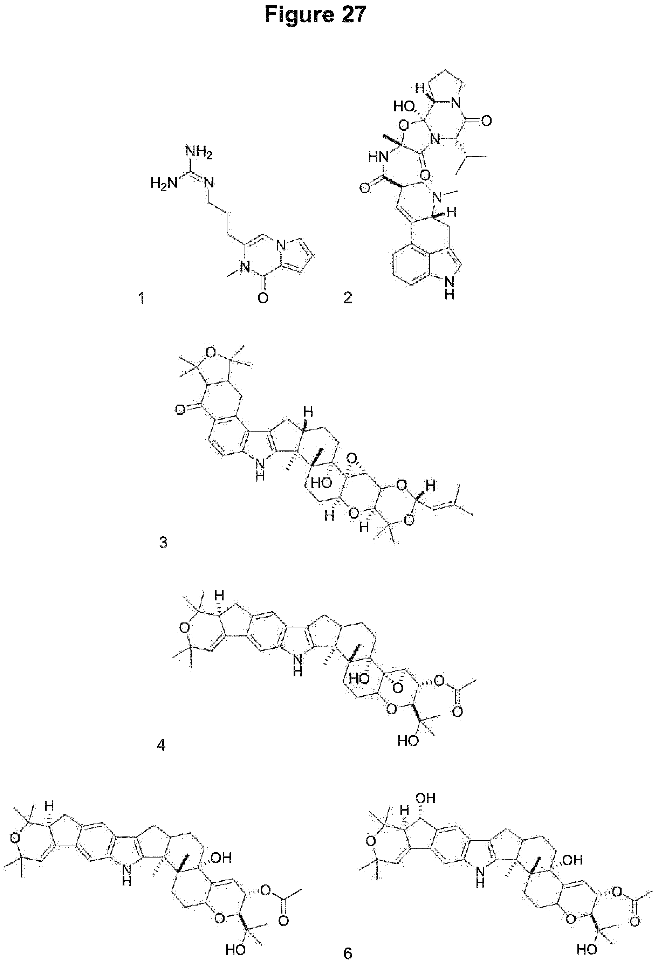

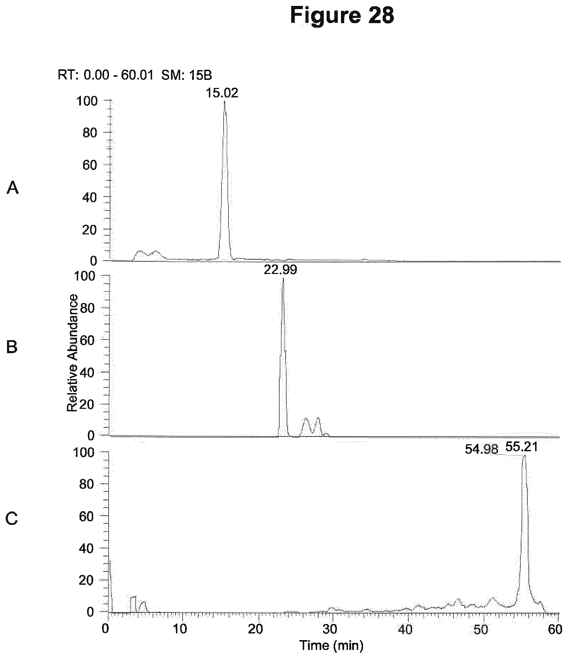

Perennial ryegrass cultivars inoculated with different endophytes were analysed for peramine (1), ergovaline (2), lolitrem B (3) and the AR37 isolated metabolites janthitrem I (4) (11,12-epoxy janthitrem G (janthitrem G (5)) by LCMS. Janthitrem G is an isomer of the previously described janthitrem F (6) and its structure was determined by NMR in the original patent describing AR37 (Latch *et al,* 2000; structures shown in Figure 27). Standards were analysed to provide reference for the perennial ryegrass analyses. The lolitrem B standard had deteriorated significantly but a peak matching the expected m/z and approximate retention time could be found (Figure 28).

Data for AR37 inoculated endophyte and NEA12-inoculated ryegrass gave comparable results. Neither contained detectable levels of peramine, ergovaline or lolitrem B. Both contained 11,12-epoxy-janthitrem G (**4**) (Figure 29). MSMS analysis of the ion *m*/*z* 646 (**4**) is shown in Figure 30. The data is a good match for that described in the original patent application.

Analysis of NEA12 was carried out in a number of perennial ryegrass cultivars. It was present to a greater or lesser extent in the majority of those examined (Table 21). No attempt was made to quantitate the amount found. A standard toxic (ST) endophyte was analysed in the same perennial ryegrass cultivars. The ST endophyte produced peramine and ergovaline but not janthitrems (Table 21). The toxin profiles for ST and NEA12 are shown in Figure 31.

**Table 21. Analysis of endophytes in different perennial ryegrass cultivars.**

| **Endophyte** | **Perennial ryegrass cultivar/inoculation event** | **alkaloids detected** |
|---|---|---|
| NEA12 | IMP04 20 | janthitrem |
| NEA12 | TOL03 18 | janthitrem |
| NEA12 | TOL03 16 | janthitrem |
| ST | TOL03 01 | peramine, ergovaline, lolitrem B |
| ST | TOL03 12 | peramine, ergovaline, lolitrem B |
| ST | IMP04 44 | peramine, ergovaline, lolitrem B |
| ST | IMP04 04 | peramine, ergovaline, lolitrem B |
| ST | BRO08 02 | peramine, ergovaline, lolitrem B |
| ST | BRO08 01 | peramine, ergovaline, lolitrem B |

| | | |
|---|---|---|
| The NEA12 endophyte appears to have the same alkaloid profile as AR37 and is distinctly different from the ST endophyte. | | |

It will be understood that the invention disclosed and defined in this specification extends to all alternative combinations of two or more of the individual features mentioned or evident from the text or drawings. All of these different combinations constitute various alternative aspects of the invention.

### References

Bouton, J. H., G. C. M. Latch, N. S. Hill, C. S. Hoveland, M. A. McCann, R. H. Watson, J. A. Parish, L. L. Hawkins and F. N. Thompson (2002) Agronomy Journal 94(3): 567 574.
Latch, G.C.M, Christensen, M.J, Tapper, B.A, Easton, H.S, Hume, D.E, Fletcher, L.R. (2000) United States Patent No. US 6111170 and references therein.
Li, X and Zhang, Y., (2002) Comparative and Functional Genomics 3: 158-160.
Tapper, B.A, Cooper, B.M, Easton, H.S, Fletcher, L.R, Hume, D.E, Lane, G.A, Latch, G.C.M, Pennell, C.G.L, Popay, A.J, Christensen, M.J. (2004) International Patent Application No. WO 2004/106487 and references therein.
Van Zijll de Jong E, Guthridge KM, Spangenberg GC, Forster JW (2003) Genome 46 (2): 277-290
Young, C.A., Bryant, M.K., Christensen, M.J., Tapper, B.A., Bryan, G.T., Scott, B. (2005) Molecular Genetics and Genomics, 274: 13-39.

## Claims

1. A substantially purified or isolated endophyte having a desired genetic and metabolic profile, said endophyte being NEA14, deposited with the National Measurement Institute under Accession No. V10/030284.

2. A plant inoculated with an endophyte according to claim 1, said plant comprising an endophyte-free host plant stably infected with said endophyte.

3. A plant, plant seed or other plant part derived from a plant according to claim 2 and stably infected with an endophyte according to claim 1.

4. Use of an endophyte according to claim 1 to produce a plant stably infected with said endophyte.

## Patentansprüche

1. Ein im Wesentlichen gereinigter oder isolierter Endophyt mit einem gewünschten genetischen und metabolischen Profil, wobei jener Endophyt NEA14 ist, hinterlegt beim National Measurement Institute unter der Zugangsnummer V10/030284.

2. Eine mit einem Endophyten gemäß Anspruch 1 inokulierte Pflanze, wobei jene Pflanze eine endophytenfreie Wirtspflanze umfasst, die stabil mit jenem Endophyten infiziert ist.

3. Eine Pflanze, ein Pflanzensamen oder ein anderer Pflanzenteil, der von einer Pflanze gemäß Anspruch 2 abgeleitet und gemäß Anspruch 1 stabil mit einem Endophyten infiziert ist.

4. Verwendung eines Endophyten gemäß Anspruch 1 zur Erzeugung einer stabil mit jenem Endophyten infizierte Pflanze.

## Revendications

1. Endophyte sensiblement purifié ou isolé ayant un profil génétique et métabolique souhaité, ledit endophyte étant le NEA14, déposé à l'Institut national de mesures sous le numéro d'enregistrement V10/030284.

2. Plante inoculée avec un endophyte selon la revendication 1, ladite plante comprenant une plante hôte sans endophyte infectée de manière stable par ledit endophyte.

3. Plante, graine de plante ou autre partie de plante dérivée d'une plante selon la revendication 2 et infectée de manière stable par un endophyte selon la revendication 1.

4. Utilisation d'un endophyte selon la revendication 1 pour produire une plante infectée de manière stable par ledit endophyte.
